# EUROPÄISCHE PATENTANMELDUNG

(11) **EP 3 461 463 A1**
(43) Veröffentlichungstag der Anmeldung: **03.04.2019**
(21) Anmeldenummer: 18000791.6
(22) Anmeldetag: 29.05.2009
(51) Int. Cl.: A61F 5/445, A61F 2/00, A61M 3/02, A61M 25/04, A61M 25/10

(54) **VORRICHTUNG ZUR STUHLDRAINAGE**

(30) Priorität: 29.05.2008 DE 102008025779; 02.06.2008 DE 102008026255; 03.11.2008 DE 102008055674; 03.11.2008 DE 102008055673; 12.02.2009 DE 102009008594; 12.02.2009 DE 102009008595
(62) Teilanmeldung aus: 09753683.3
(71) Anmelder: Advanced Medical Balloons GmbH, 68753 Waghäusel (DE)
(72) Erfinder: Göbel, Fred, Dr. med., 67346 Speyer (DE)
(74) Vertreter: Küchler, Stefan

(57) **Zusammenfassung**

Die Erfindung richtet sich auf eine Vorrichtung zum Abdichten einer natürlichen oder künstlichen Öffnung des Dickdarms oder Colons bzw. Enddarms oder Rektums eines Patienten sowie zum Verschließen derselben und/oder zur Entfernung von Stuhl daraus, insbesondere zur intermittierenden Irrigation und/oder zur kontinuierlichen Drainage, vorzugsweise in ein externes, beutelartiges Auffanggefäß, wobei die Vorrichtung (i) untergliedert ist in einen radial erweiterten Bereich als intrarektales Segment zum Einführen in das Rektum sowie einen demgegenüber verjüngten Bereich als transanales Segment, der während des Gebrauchs zumindest bereichsweise außerhalb des Rektums verbleibt, und (ii) einen aufblasbaren Ballon von etwa ringförmiger Struktur umfasst, der aus einem flächigen, in sich umgestülpten Schlauchabschnitt (8) gebildet ist und ein oder mehrere, mit Fluid befüllbare Kompartimente aufweist; umfassend ein mit einem Kompartiment versehenes, intrarektales Ballonsegment, das kein gemeinsames Kompartiment mit dem besagten transanalen Segment besitzt, wobei das nach distal gerichtete Ende des ausgeformten Ballons durch das Lumen des intrarektalen Ballonsegmentes gestülpt und im Lumen des nach proximal gerichteten Ballonendes weitergeführt ist und im Bereich des proximalen Ballonendes mit jenem verbunden ist, um das befüllbare Kompartiment des intrarektalen Ballonsegmentes abzuschließen.

## Beschreibung

Die Erfindung richtet sich auf eine Vorrichtung zum Abdichten bzw. Verschließen einer Öffnung des Dickdarms (Colon) bzw. Enddarms (Rektum) eines Patienten sowie ggf. zur kontinuierlichen und/oder intermittierenden Drainage bzw. Entfernung von Stuhl daraus, vorzugsweise in ein externes, beutelartiges Auffanggefäß, wobei die Vorrichtung untergliedert ist in einen radial erweiterten Bereich als intrarektales Segment zum Einführen in das Rektum sowie einen demgegenüber verjüngten Bereich als transanales Segment, der während des Gebrauchs zumindest bereichsweise außerhalb des Rektums verbleibt; und wobei die Vorrichtung ferner einen aufblasbaren Ballon von etwa ringförmiger Struktur umfasst, der aus einem flächigen, in sich umgestülpten Schlauchabschnitt gebildet ist und ein oder mehrere, mit Fluid befüllbare Kompartimente aufweist.

Vorrichtungen zur kontinuierlichen Drainage oder intermittierenden Entfernung von Stuhl aus dem Dickdarm (Colon) bzw. Enddarm (Rektum) eines Patienten in ein externes, beutelartiges Auffanggefäß, insbesondere auch durch einen rektal applizierten Einlauf von Spülflüssigkeit, sind seit einigen Jahren bekannt.

Zur Ableitung bzw. zum Auffangen von Stuhl werden bei immobilen, nicht kooperativen Patienten vorzugsweise so genannte Fäkalkollektoren verwendet. Diese stellen beutelartige Strukturen dar, die in der Analfalte direkt über der Analöffnung aufgeklebt werden. Zwar ist die prä-anale Verklebung in der Regel über kürzere Anwendungsintervalle ausreichend haftend und dichtend, wegen des anhaltend feuchten und chemisch aggressiven Milieus im Verklebungsbereich sind jedoch häufig Mazerationen der exponierten Hautpartien zu beobachten.

Alternativ sind so genannte Darmrohre in Gebrauch, die über die Analkanal in den Enddarm eingeführt werden. Wegen des einhergehenden intra-rektalen Verletzungsrisikos sowie der permanenten Dilatation und damit möglichen Schädigung des Schließmuskels werden Darmrohre in der Regel nur passager zur Stuhlableitung verwendet.

Systeme zur weitgehend atraumatischen, langfristig anwendbaren Stuhlableitung (indwelling fecal drainage), wie sie kürzlich von der Fa. Zassi Medical Evolutions Inc., Florida, USA und ConvaTec, New Jersey, USA, vorgestellt wurden, entsprechen in ihrem Aufbau konzeptionell Blasenkathetern zur kontinuierlichen Drainage von Urin. Über ein ballontragendes Katheter-Element wird der Urin aus der Blase abgeleitet und fließt über einen angeschlossenen Drainageschlauch in einen Sammelbeutel. Das Ballon-Element dient dabei vorwiegend der Verankerung des Katheters in der Blase. Darüber hinaus gewährleistet es eine gewisse Dichtungsfunktion, die verhindert, dass Urin am Katheterschaft vorbei durch die Harnröhre abfließt. Die bekannten Stuhldrainage verfügen analog über einen verankernden und abführenden ballontragenden Kopfteil, sowie über ein sich anschließendes Schlauchelement, welches in einen Sammelbehälter mündet.

Moderne Stuhldrainagesysteme, wie die oben genannten, gewährleisten über die reine Drainageleistung hinaus die aktive Führung der Stuhlausscheidung des Patienten im Sinne eines Stuhlmanagements (fecal management) durch den Therapeuten.

Das Konzept des Stuhlmanagements beinhaltet die Option einer höher- bzw. großvolumigen, anal applizierten colo-rektalen Irrigation. Großvolumige Einläufe in den Dickdarm werden bereits seit Jahrzehnten von Patienten mit chirurgisch angelegtem Dickdarmausgang zur aktiven Führung der Ausscheidung angewandt. Die in das Colon einlaufende Spülflüssigkeit mobilisiert den Dickdarminhalt, löst die peristaltische Tätigkeit des Darmes aus, wodurch sich das Colon, quasi vom Anwender initiiert oder getriggert, weitgehend entleert.

In entsprechender Weise kann bei immobilen, nicht kooperativen Langliegern durch einen großvolumigen trans-analen Einlauf Darminhalt mobilisiert und drainiert werden. Die Defäkation wird so vom Therapeuten kontrolliert und geführt. Bei entsprechender Anwendung kann ein stuhlfreies, quasi kontinentes Intervall von ein bis zwei Tagen hergestellt werden.

Colorektale Einläufe (die sog. colorektale Irrigation) wurden in der klinischen Praxis bislang vorwiegend zur Mobilisation von Stuhl bei vorliegender Verstopfungssymptomatik bzw. Darmentleerungsstörungen angewandt.

Seit etwa einem Jahrzehnt wird das Prinzip der colorektalen Irrigation allerdings mit großem Erfolg auch bei mobilen, selbständigen inkontinenten Patienten eingesetzt, deren Stuhlentleerung (Defäkation), durch verschiedenste Ursachen bedingt, nicht mehr der willkürlichen Kontrolle unterliegt. Das über den Anus in das Rektum bzw. Colon irrigierte Flüssigkeitsvolumen wirkt bei diesen Patienten im Sinne einer Stimulation der Darmperistaltik, mit dem Ziel einer möglichst vollständigen Entleerung des Dickdarmes. Für den Patienten resultiert ein stuhlfreies Intervall, in dem trotz manifester Verschlußinsuffizienz des Anus keine unkontrollierte Passage von Stuhl über den Anus erfolgen kann. Für eine ausreichende Spülung ist entscheidend, dass durch dad eingelaufene Volumen sowohl Stuhl im Colon mobilisiert, als auch die Peristaltik bzw. der Defäkationsreflex ausgelöst werden. Nach der Instillation des Einlaufvolumens schließt sich in der Regel ein Intervall von mehreren Minuten an, in dem sich der Dickdarm still verhält, bis schließlich peristaltische Kontraktionen einsetzen und das Colon bzw. Rektum beginnen den Darminhalt auszutreiben. Um das erforderliche Einlaufvolumen im Darm halten zu können bis reflexgesteuerte Austreibung einsetzt, sind trans-anal anzuwendende Irrigationshilfen häufig mit einem dichten Element ausgestattet. Dieses stellt sich im terminalen Rektum in der Regel als prall gefüllter Ballon dar, welcher einem rohr-ähnlichen Einführelement aufsitzt und in der Wartephase bis zum Einsetzen der Dickdarmperistaltik das Austreten von Spülflüssigkeit durch den Anus vermeiden soll.

Problematisch sind momentan verfügbare Stuhldrainage- und Irrigations-Systeme in vielerlei Hinsicht:
Bei angestrebten Liegezeiten der Drainage von bis zu vier Wochen, sind druckbedingte Verletzungen im Bereich des terminalen Rektums (Ulcera) sowie Läsionen im Bereich des Anus (Fissuren) nicht auszuschließen. DruckUlcera sind in der Regel die Folge prall gefüllter, die betreffende Vorrichtung im Rektum verankernder Ballonkörper, welche den rektalen Strukturen dauerhaft anliegen und deren Durchblutung einschränken. Die bekannten Systeme besitzen Ballons, die im nicht befüllten Zustand nur partiell ausgeformt sind, und durch Beaufschlagung mit ca. 100 mbar auf ihr Arbeitsmaß aufgedehnt werden, um so ihre Ankerfunktion zu gewährleisten. Anale Fissuren hingegen resultieren in der Mehrzahl aus der fortwährenden Reibung, die der in der Regel kollabierte, mehr oder weniger rigide, in Faltung liegende trans-anale Drainageabschnitt der Vorrichtung im Anus verursacht.

Des Weiteren bieten die bekannten Systeme vor allem bei dünnflüssigem Stuhl bzw. bei einem höhervolumigen Einlauf von Spülflüssigkeit ins Colon keine wirklich ausreichende Dichtungsleistung, was fortwährendes Einstuhlen zur Folge hat und die engmaschige Reinigung der Analfalte erfordert.

Bisherige Systeme neigen dazu, bei Zug am Katheter aus dem Rektum des Patienten heraus zu gleiten. Das so genannte "Schlupfen" ist zum Teil eine Folge der Dehnungs- und Verformungseigenschaften der aktuell verwendeten Materialien, vorrangig Silikon. Die hohe Compliance von Silikon gestattet keine ausreichende Formstabilität des Ballons und führt bei axialem Zug an der Drainage zu einer zunehmenden tropfenförmigen Verformung und Verjüngung des Ballonankers, bis dieser soweit verformt ist, das er durch die zu dichtende Einführöffnung nach außen luxiert.

Herkömmliche elastisch aufzudehnende Ankerballons verfügen zudem nicht über einen ausreichend großen Ballondurchmesser um auf dem Rektumboden deckend aufzuliegen und so den bestmöglichen Ankereffekt gegen die Analöffnung zu erreichen.

Um eine möglichst schonende dauerhafte Katheterisierung des besonders empfindlichen trans-analen Abschnittes zu ermöglichen, wird bei herkömmlichen Systemen das entsprechende Segment der Drainage möglichst dünnwandig ausgeführt, um in seiner Konsistenz möglichst weich und gewebefreundlich zu erscheinen. Mit einer solchen dünnwandigen Ausführung des trans-analen Segmentes geht in der Regel begleitend die Neigung zur Torsion bzw. Verwindung über die Längsachse des trans-analen Segmentes einher, wodurch der Stuhlabfluß entscheidend behindert bzw. unmöglich wird.

Silikone sind überdies nicht geruchsdicht und geben in der Regel nach einigen Tagen Liegedauer einen faulig fäkalen Geruch an die Patientenumgebung ab. Silokonoberflächen sind zudem häufig uneben und führen wegen der kraterartigen Oberfläche daher zur weitgehend reinigungsresistenten Verschmutzung.

Bislang verfügbare Systeme aus Silikon wiesen zudem einen relativ komplexen Aufbau auf, und werden aus einer Vielzahl von Komponenten, in kostenintensiver manueller Arbeit verklebt.

Neuere Irrigationsgeräte, wie z.B. das System Peristeen Anal Irrigation der Fa. Coloplast, Dänemark, sind für die spezifischen Anwendungsanforderungen von Patienten entworfen, die die Spülung bei sich selbst durchführen bzw. die Ausscheidung von Stuhl selbst führen und kontrollieren. Die aktuell verfügbaren Systeme bieten zwar einen gewissen Handhabungskomfort, auf einige, für den Anwender wesentliche Anforderungen im praktischen Gebrauch gehen sie jedoch nicht ein.

Ferner sind die verfügbaren Spülsysteme zur Erreichung kontinenter Phasen im Wesentlichen für den selbstversorgenden Patienten ausgelegt. Für bettlägrige nicht ausreichend kooperative inkontinente Patienten schließt sich deren Anwendung weitgehend aus.

Mangelhaft sind in der Regel vor allem die Dichtungseigenschaften zum Anus hin, was in der Mehrzahl der Fälle auf eine nicht ausreichend symmetrische Aufdehnung des rektal dichtenden Ballonelementes zurückzuführen ist. Die unzuverlässige geometrische Ausdehnung ist bedingt durch die Verwendung hoch-volumendehnbarer Ballonmaterialien wie z.B. Latex, Silikon oder künstlichen Kautschuk-Sorten, die als schlauchartiges, nicht präformiertes Element auf dem einführenden Schaft aufgebracht werden und sich bei Befüllung unter Druck zu sphärischer Form entwickeln. Dabei entstehende bauchartige Ausstülpungen der Ballonhülle, so genannte Herniationen, können derart ausgeprägt sein, dass das Schaftelement in die Hernie hinein verlagert wird, und nicht mehr im Zentrum des Ballons liegt. Bei einer derartigen Konfiguration von Schaft und Ballon eine zentrale Auflage des Ballons über dem Anus auf dem Rektumboden unmöglich werden, und so eine Leckage verursachen, die der Patient oft nur durch fortwährende manuelle Korrektur der Lage des Katheter-Schaftes im Anus bzw. der Lage des Ballons im Rektum beheben kann.

Ein weiteres Problem kann für Patienten die komfortable Einbringung des Einlaufs selbst sein. Verfügbare Systeme, wie z.B. das Persisteen System, arbeiten z.B. auf der Basis einer pneumatischen Förderung von Spüllösung, die über eine Handpumpe bedient werden kann. Von selbstversorgenden Patienten werden ebenfalls elektrische Pumpsysteme verwendet. Vor allem bei der kontinuierlichen Drainage kommen jedoch schwerkraft-angetriebene Einlaufsysteme zur Anwendung. Systeme, die den Handhabungserfordernissen aller Irrigations- und Drainagekonzepte gerecht werden, sind jedoch nicht auf dem Markt.

Beginnt die peristaltische Austreibung von Darminhalt, wird bei den aktuellen System in der Regel der Einlaufkatheter vom Patienten aus dem Anus entfernt. Der ausgetriebene Inhalt entleert sich dann mehr oder weniger abrupt und kann zur Verschmutzung des Patienten bzw. der Patientenumgebung führen. Konventionelle Einlaufsysteme, bei denen über einen Beutel zuerst die Lösung in den Darm appliziert, und anschließend der ausgetriebene Darminhalt wieder zu diesem Beutel zurückgeführt wird, können das peristaltisch geförderte Volumen jedoch in vielen Fällen nicht aufnehmen, und es kommt zur teils spritzenden Leckage am analen Einlaufelement vorbei.

WO 2006/010556 A1 beschreibt eine gattungsgemäße Vorrichtung zum Verschließen eines natürlichen oder künstlichen Darmausgangs, umfassend einen aufblasbaren Ballon mit einer etwa torusförmigen Struktur, gebildet aus einem flächigen, in sich umgestülpten Schlauchabschnitt, dessen beiden Enden etwa koaxial ineinander verlaufen und mit (je) einer Hülse verbunden sind, wobei die äußere Lage des umgestülpten Schlauchabschnitts einen radial erweiterten, patientenproximalen Bereich zum Einführen in das Rektum aufweist (intra-rektaler Bereich) sowie einen demgegenüber verjüngten, patientendistalen Bereich, der während des Gebrauchs zumindest bereichsweise außerhalb des Rektums verbleibt (trans-analer Bereich). Der trans-anale Teil wird aus zwei konzentrischen Folienlagen aufgebaut, wobei die konzentrischen Schlauchfolien durch punktuelle Verbindungen dauerhaft strukturell miteinander verbunden sein können. Das so zwischen den Lagen definierte Kompartiment kommuniziert jedoch frei mit dem Lumen des intrarektalen Ankerballons. Beide Räume werden über eine einzige Zuleitung befüllt. Dies hat zur Folge, dass infolge eines beständigen Druckausgleichs zwischen beiden Bereichen das Volumen eines Bereichs nicht gezielt beeinflußt werden kann, worunter bspw. die Verankerungswirkung des intrarektalen Bereichs leiden könnte.

Das die Erfindung initiierende Problem besteht demnach darin, die geschilderten Nachteile bei der Anwendung konventioneller Systeme zum Abdichten bzw. Verschließen des Dick- und/oder Enddarms sowie ggf. zur intermittierenden, groß-volumigen colorektalen Irrigation und/oder zur kontinuierlichen Stuhldrainage zu vermeiden.

Die Lösung dieses Problems gelingt dadurch, dass bei einer gattungsgemäßen Vorrichtung der intrarektale Ballonabschnitt in keiner funktionellen oder räumlichen Verbindung mit dem transanalen Ballonabschnitt steht; vielmehr weist das intrarektale Segment (1) ein mit einem Fluid befüllbares, ballonförmiges Kompartiment auf, wobei das besagte intrarektale Ballonsegment (1) und das besagte transanale Segment (2) kein gemeinsames Kompartiment besitzen, und wobei das nach distal gerichtete Ende des ausgeformten Ballons (8) durch das Lumen des intrarektalen Ballonsegmentes (1) gestülpt und im Lumen des nach proximal gerichteten Ballonendes weitergeführt ist und im Bereich des proximalen Ballonendes mit jenem verbunden ist, um das befüllbare Kompartiment des intrarektalen Ballonsegmentes (1) abzuschließen.

Zwar könnte in der vorliegenden Erfindung das trans-anale Segment ebenfalls ganz oder teilweise aus den nach proximal bzw. distal gerichteten Fortsetzungen des intra-rektalen Anker-Ballons hervorgehen, beide Segmente, das intra-rektale und trans-anale, sind jedoch strukturell und funktionell sowie insbesondere räumlich voneinander getrennt und kommunizieren nicht miteinander und werden bei Bedarf getrennt voneinander befüllt und/oder entleert.

Da im Gegensatz zur WO 2006/010556 A1 bei der vorliegenden Erfindung der intra-rektale Ballonanteil in keiner funktionellen oder räumlichen Verbindung noch in einer sonstigen, kommunizierenden Verbindung mit dem trans-analen Segment steht, gibt es keine Verschiebung des/der Füllfluide zwischen beiden Bereichen. Demzufolge weisen die beiden Bereiche eine erheblich gesteigerte Formstabilität auf. Bei Bedarf könnte bspw. der Druck und/oder das Füllvolumen in einem der beiden Kompartimente unabhängig vom Zustand des anderen verändert werden.

Bevorzugt verzichtet die Erfindung bei der Gestaltung des ano-rektalen, dichtenden Ballonelements auf stark volumendehnbare Materialien mit unzuverlässiger Geometrie bei der Aufdehnung von einem kleinerdimensionierten Grundzustand in den Arbeitszustand. Silikon als bislang in der klinischen Anwendung verwendetes Basismaterial wird vorzugsweise durch Materialien mit geringer Volumendehnbarkeit (Compliance) wie z.B. Polyurethan (PUR) ersetzt. Insbesondere bei der Gestaltung der rektal dichtenden Ballonelemente werden bevorzugt vollständig ausgeformte, dünnwandige Ballonstrukturen aus beispielsweise Polyurethan (PUR) oder Materialien mit ähnlichen Elastizitäts- und Festigkeitseigenschaften verwendet. Die Erfindung nutzt die Option zur vollständigen dimensionalen, insbesondere dreidimensionalen Ausformung der Folienelemente auf das Arbeitsmaß sowie die Möglichkeit der strukturell detaillierten Ausformung von PUR-Ballonfolien im blow-molding-Verfahren. Die durch PUR gegebene Option zur vollständigen und strukturell detaillierten Ausformung dünnwandigster Ballonkörper im blow-molding Verfahren soll neben überlegener Funktionalität hinsichtlich Ankerfunktion und Dichtung zur kostengünstige Herstellung und Montage der Drainage- und/oder Irrigationsvorrichtung beitragen. Die Anzahl der Komponenten, die für den Aufbau der im bzw. nahe am Patienten zu liegen kommenden Kopfeinheit der Vorrichtung erforderlich ist, soll reduziert werden. Montage und dauerhafte, insbesondere mechanische Verbindung der Komponenten soll durch weitgehende Steckbarkeit und Rastung bzw. Verrastung der Einzelelemente vereinfacht und ökonomisiert werden. Die beschriebenen Montageoptionen gehen dabei auf die besonderen mechanischen membran-artigen Eigenschaften extrem dünnwandiger Ballonfolien ein. Des Weiteren stellt die Erfindung funktionell besonders vorteilhafte Einzelkomponenten bzw. Segmentgestaltungen vor, die in Hinsicht auf langfristige Gewebeverträglichkeit, Ankereffizienz, kontinuierliche Dichtungsleistung und Dichtung bei dünnflüssigem Stuhl bzw. appliziertem großvolumigem Einlauf einen Vorteil gegenüber dem Stand der Technik bieten.

Im Gegensatz zu herkömmlichen Systemen, bei denen das dichtende Ballonelement auf volumendehnbaren Materialien wie Silikon oder Latex basiert, verwendet die Erfindung für den Ballon bevorzugt Polyurethan (PUR) als Basismaterial oder ein Material mit ähnlichen technischen Elastizitäts- und Festigkeitseigenschaften.

Die Erfindung sieht weiterhin vor, dass der intra-rektale sowie der trans-anale Drainageteil bevorzugt aus PUR gefertigt sind. Beide Drainageteile werden hier aus einem einzigen durchgängigen, in sich vollständig rückgestülpten Ballonelement hergestellt. Das nach distal gerichtete Ende des ausgeformten Ballonrohlings wird durch das Lumen des intra-rektalen Ballonsegmentes gestülpt, und im Lumen des nach proximal gerichteten Ballonendes bis zu dessen Ende weitergeführt. Beide Ballonenden werden parallel zueinander, auf etwa gleicher Höhe, auf einem vor dem Anus liegenden Konnektorelement fixiert. Auch weist der intra-rektale Ankerballon im rückgestülpten, montierten Zustand eine logenartige Präformation zur Aufnahme eines separat gefertigten Trichterelementes auf.

Die Erfindung beschreibt in einigen Ausführungsformen ein mit einem Füllmedium beaufschlagbares trans-anales Kompartiment auf der Basis konzentrischer Schlauchelemente. Dieses ist jedoch vom intra-rektalen Ballon separiert. Beide Kompartimente werden separat befüllt und stellen funktionell eigenständige Einheiten dar.

Die Trennung der beiden Segmente wird in der Erfindung vorzugsweise durch einen dauerhaften, dichtenden Abschluß der Hülle des intra-rektalen Ballonsegmentes auf oder mit einem Trichterelement erreicht. Sie kann jedoch im Bereich des proximalen Endes des intra-rektalen Ballons auch durch direkte Verbindung der Ballonhülle mit der Oberfläche von Anteilen des trans-analen Segmentes hergestellt werden.

Die Erfindung beschreibt darüber hinaus den trans-analen Anteil der Vorrichtung als nicht ausschließlich zwei-lagig ausgeführt, sondern geht auch auf ein-lagige Varianten dieses Segmentes ein.

Als Ausführungsvarianten werden ebenfalls Ausgestaltungen des trans-analen Segmentes beschrieben, die neben einer ein- oder zwei-lagigen Schlauchlage, ein zusätzliches funktionelles Element aufweisen, welches vorrangig der axialen Torsion bzw. dem verwindungsbedingten Verschluss dieses Abschnittes entgegenwirken soll.

Während in WO 2006/010556 A1 ein passagerer Verschluß des Drainagelumens durch einen im intra-rektalen Trichter entfaltenden Verschlußballon hergestellet werden kann, wird ein solcher Verschluß in der vorliegenden Erfindung im Bereich des trans-analen Drainage-Anteils ermöglicht. Hierbei wird nicht nur das Drainagelumen verschlossen, sondern durch das sich bei Befüllung zusätzlich nach radial ausdehnende Segment auch simultan gegen den Analkanal hin gedichtet.

Das in WO 2006/010556 A1 ebenfalls vorgestellte, vollständig bzw-. optional auch residual bemessene intra-rektale Ballonsegment findet sich in der Erfindung ebenfalls. In der vorliegenden Erfindung wird dieses in der praktischen Anwendung vorzugsweise mit einem definierten Volumen befüllt, welches bewusst kleiner gewählt wird als das Volumen des frei entfalteten (befüllten, aber drucklosen) vollständig ausgeformten Ballons. Das intra-rektale Ballonsegment liegt somit dem Rektumboden als schlaffer, nur teilweise gefüllter Ballonkörper auf. Er schmiegt sich der jeweiligen Morphologie unter allseitiger Einfaltung seiner Hülle dynamisch an. Er kann so dauerhaft, mit geringst möglicher auf das Rektumgewebe wirkender Kraft bzw. geringer Wahrscheinlichkeit Gewebeschäden zu verursachen, den individuellen Raum komplett ausfüllend, platziert sein. Der Fülldruck, der zur Erreichung einer ausreichenden Ankerfunktion der Drainage im Rektum erforderlich ist, kann im Idealfall nicht größer als der dort herrschende physiologische Druck sein. Bei steigendem colo-rektal eingebrachten Spülvolumen folgt der Druck im Ballon synchron dem von der über dem Ballon aufgebauten Flüssigkeitssäule. Die besonderen Dichtungsfähigkeiten der verwendeten membranartigen Ballonfolie machen zur Erreichung ausreichender Dichtung während der Irrigation keine vom Anwender kontrollierte Fülldruckerhöhung zwingend erforderlich. Sie kann jedoch bei besonders problematischer Anatomie nötig sein.

Während beim Stand der Technik eine derartige Absenkung des Fülldruckes auch gleichzeitig die Entfaltungscharakteristika und damit Drainageeigenschaften des trans-analen Segmentes bestimmt, ist dies bei der vorliegenden Erfindung nicht der Fall. Beide Kompartimente können in ihrer Kraftentwicklung auf das ihnen anliegende Gewebe getrennt reguliert werden.

Wirkt eine axial bzw. nach proximal gerichtete Zugkraft auf einen derartigen voll ausgeformten, nur partiell befüllten Ballon, unabhängig ob dieser residual über die anzunehmenden rektalen Maße hinaus präformiert ist oder im frei entfalteten Zustand eine Dimension annimmt, die kleiner ist als der auszufüllende rektale Raum, entwickelt sich nach dem Prinzip "actio = reactio" ein Ballonfülldruck, der exakt derjenigen Kraft entspricht, die von proximal am Anker-Ballon wirkt. Lässt die jeweilige Zugkraft nach, kehrt der Ankerballon wieder auf seinen niedrigen Initialdruck zurück. Das initial eingebrachte Füllvolumen kann beispielweise bei 70-80% des frei entfalteten, präformierten Volumens liegen, und vom Anwender so portioniert als einmalige Spritzenfüllung in den Ballon über die Füllleitung eingebracht werden.

Bei Verwendung nicht oder nur gering volumen-dehnbarer Materialien wie z.B. PUR der Spezifikation Pellethane 2363 80A, Dow Chemical Corp., ist gewährleistet, das der Ankerballon unter anliegender Zugwirkung und damit resultierender Steigerung des Fülldruckes seine bei der Herstellung angenommene Geometrie und Dimension annimmt, diese jedoch, wegen seiner materialbedingten limitierten Verformbarkeit (Compliance) nicht soweit deformiert werden kann, das sich der Ballon nach distal tropfenförmig ausstreicht und schließlich durch die Analöffnung schlüpft, wie dies bei hochvolumendehnbaren Materialien, wie z.B. Silikon oder Latex zu erwarten wäre und in der täglichen Anwendung auch zu beobachten ist.

Das trans-anale Segment besteht bei der Erfindung vorzugsweise aus den verlängerten Enden des intra-rektalen Ballons, kann jedoch auch aus separat gefertigten Schlauchelementen bzw. aus einem separat gefertigten Schlauchelement gefertigt werden, die mit dem intra-rektalen Ballon an dessen Enden jeweils fest verbunden sind. Die Schlauchenden werden optional mit einem zusätzlichen rohr- oder schlauchartigen Element verbunden bzw. auf diesem dauerhaft aufgebracht. Dieses Element kann sich optional in das intrarektale Segment hinein verlängern und dort eine den Stuhl aufnehmende und bahnende, trichterartige Form ausbilden. Selbst wenn das trans-anale Segment in diesem Fall doppel- oder dreiwandig ausgeführt ist, bildet es dennoch kein mit dem intra-rektalen Segment gemeinsam befüllbares Kompartiment.

Zur Stabilisierung des trans-analen Segmentes kann zusätzlich ein, das Trichterelement mit dem Konnektionselement vorzugsweise durchgängig verbindendes Rohrelement verbaut werden. Zur Vermeidung der lumeneinengenden Torsion des trans-analen Segmentes wird dieses vorzugsweise als sich elastisch selbst aufrichtendes Schlauchelement oder als radial verformbares, sich dem Analkanal mit moderater Spannung anschmiegendes, und selbst öffnendes Rohrgeflecht ausgeführt.

Verfügt das trans-anale Segment über eine doppelwandige Ausführung, kann die sich zwischen Trichter- und Konnektorelment darstellende Kammer über eine entsprechende Zuleitung von außerhalb des Patienten entweder evakuiert oder mit einem Füllmedium, vorzugsweise Luft, befüllt werden.

Wird der Raum zwischen den beiden konzentrischen Schlauchlagen evakuiert, kommen diese dicht aneinander zu liegen und stellen sich, voneinander nicht trennbar, als eine einzige Lage dar. Das zentrale Drainagelumen ist dann maximal geöffnet.

Wird der Raum zwischen den konzentrischen Schlauchlagen befüllt, trennen sich die beiden Lagen in der Art, das sich die innere Lage in das Drainagelumen hinein entfaltet und dieses dichtend verschließt. Die äußere Lage hingegen dehnt sich nach radial zur Wandung des Analkanals hin aus und führt so zu dessen Dichtung.

Die Befüllung und Entfaltung des trans-analen Segments in der beschriebenen Weise ist besonders vorteilhaft für die großvolumige colo-rektale Irrigation, die zum Ziel hat, ein möglichst großes, hohe Colonabschnitte erreichendes Spülvolumen in den Darm einlaufen zu lassen und dabei ein Entweichen der Spüllösung durch das Drainagelumen bzw. durch den Anus, am trans-analen Drainagesegment vorbei, zu vermeiden.

Die Dichtungsleistung bei appliziertem großvolumigem Einlauf wird durch die Verwendung vollständig ausgeformter, membranartiger Ballonfolien verbessert, die sich der individuellen rektalen Anatomie unter geringst möglichem Fülldruck bestmöglich anpassen.

Zum anderen soll durch eine Option zur zusätzlichen Dichtung über das transanale Segment hinweg das Auslaufen von Spülflüssigkeit im Verlaufe des Irrigationsvorgangs verhindert werden.

Die Erfindung beschreibt weiter die effiziente Aufnahme und Drainage von Stuhl- und Spülflüssigkeit über ein großlumiges Drainagelumen, welches durch ein intra-rektales Trichterelement im intra-rektalen Dichtungsballon führt, sowie über ein sich an den Trichter anschließendes, durch den Analkanal hindurch verlaufendes großlumiges Schlauchelement, welches direkt in ein Beutelgefäß mündet.

Die Spülflüssigkeit und darin enthaltene Anteile geformten Stuhls, können so, ohne vorherige Entfernung der Einlaufvorrichtung aus dem Rektum, bei intrarektal verbleibender Vorrichtung, unabhängig von einer den Stuhl aufnehmenden Toilette oder einer entsprechenden Einrichtung, auch bei bettlägerigen oder nicht ausreichend kooperativen Patienten abgeführt werden.

Bedingt durch die besonderen, weitgehend atraumatischen Charakteristika der verwendeten Ballonfolien der intra-rektalen bzw. trans-analen Anteile der Vorrichtung, kann das Einlaufelement auch prolongiert im Patienten verbleiben und muss nicht notwendigerweise nach jeder Anwendung entfernt werden.

Die im Patienten verbleibende Vorrichtung kann in den zwischen den Einläufen liegenden Intervallen eine rektal-anal dichtende Funktion ausüben. Der Katheter kann an seinem proximalen Ende außerhalb des Anus hierzu flüssigkeitsdicht aber gasdurchlässig verschlossen werden. Wird am proximalen Ende ein beutelartiges Element angeschlossen, kann im Intervall auch rektal anflutendes Sekret oder Stuhl drainiert werden.

Ebenfalls beschrieben werden für die Anwendung bei immobilen bzw. nicht kooperativen Patienten vorteilhafte Auffanggefäße, die zum einen die Aufnahme der Spülflüssigkeit, als auch die Förderung in den Patienten, als auch die Drainage für den Patienten erleichtern.

Die Erfindung beschreibt ferner ein Fördersystem für die Applikation der Spülflüssigkeit; dieses kann den jeweiligen Fertigkeiten der Patienten angepasst sein.

Der Einlauf von Spülflüssigkeit kann beispielsweise schwerkraft-getrieben erfolgen. In diesem Falle bringt der Therapeut oder Patient ein definiertes Volumen von Spüllösung auf ein geeignet hohes Niveau über dem Einlaufpunkt. Der Zulauf erfolgt spontan.

Als treibende Kraft für den Einlauf ist ebenfalls eine manschettenartige Vorrichtung denkbar, wie sie beispielsweise für die druck-angetriebene, schnelle intra-vaskuläre Infusion verwendet wird. Ein beutelartiger Behälter mit Spüllösung wird dabei in das offene zentrale Lumen einer mit Druckluft beaufschlagbaren Manschette gesteckt und von dort aus bei steigendem Fülldruck in der Manschette quasi ausgedrückt bzw. zum Patienten hin entleert. Der erforderliche Druck wird in der Regel mit einer Handpumpe erzeugt. Neben einem gasförmigen Medium zum Druckaufbau in der Manschette sind ebenfalls flüssige Medien, wie z.B. Leitungswasser denkbar.

Denkbar ist ebenfalls ein Vakuumantrieb mittels Wasserstrahlpumpe. Der die Spüllösung aufnehmende Beutel wird dabei in einen diesen allseitig umgebenden Beutel verbaut, der einem Vakuum ausgesetzt wird, welches beispielsweise am Wasserhahn durch Verwendung einer Wasserstrahlpumpe erzeugt werden kann.

Neben der kontinuierlichen Drainage von Stuhl aus dem Rektum eines Patienten ist die Anwendung der in der Erfindung beschriebenen Kopfeinheit, bestehend aus intra-rektalem Ankerballon und trans-analem Segment auch für die intermittierende Anwendung bei inkontinenten bzw. nur bedingt kooperativen Patienten denkbar.

Bekannte Einlauf-Vorrichtungen, wie z.B. des System Peristeen Anal der Fa. Coloplast, Dänemark, weisen rektal dichtende Ballonkomponenten auf, die auf einem Katheterschaft aufsitzen und elastisch auf ihr Arbeitsmaß aufgedehnt werden. Das relativ kleine Lumen des Einlaufkatheters gestattet zwar den zügigen Einlauf von Spülflüssigkeit, erlaubt in der Regel jedoch nicht den freien Auslauf von mit Stuhlpartikeln beladener Spülflüssigkeit. Der Katheter muss daher in der Regel bei einsetzender Peristaltik gezogen werden, der Einlauf entleert sich dann via naturalis.

Wünschenswert wäre jedoch eine Einlauf-Anwendung, bei der die Entleerung problemlos über den rektal platzierten Katheter erfolgen kann. Vorteilhaft wäre dies beispielsweise bei bettlägerigen Patienten, die im Bett liegend komfortabel und hygienisch gespült und in eine kontinente Phase überführt werden können. Das in der Erfindung beschriebene trans-anale Drainage-Lumen, mit seinem Durchmesser von bis zu 3 cm, gestattet den freien Abgang auch geformter Stuhlanteile.

Die zuvor beschriebenen Ausführungen der Kopfeinheiten der Drainagevorrichtung kommen sämtlich für die Verwendung in diesem Kontext in Frage. Anstatt des Drainageschlauches kann direkt, oder über ein kurzes Schlauchzwischenstück, an den Konnektor ein beutelartiges Auffanggefäß angeschlossen werden.

Bedingt durch das große Drainagelumen sind ebenfalls sogenannte Hebe- und Senkeinläufe denkbar, bei denen das Spülvolumen zwischen Beutel und Patient sozusagen pendelt.

Alternativ, bei selbst-therapierenden Patienten, die auf der Toilette sitzend abführen, kann ein kurzer, tüllen-artiger Schlauch oder Folienschlauch angeschlossen sein.

Zwar können die beiden Enden des in sich umgestülpten Schlauchabschnitts etwa koaxial ineinander verlaufen und eventuell mit (je) einer Hülse verbunden sein; bei einer bevorzugten Ausführungsform der Erfindung ist dies jedoch nicht der Fall.

Weitere Merkmale, Eigenschaften, Vorteile und Wirkungen auf der Basis der Erfindung ergeben sich aus der folgenden Beschreibung verschiedener Ausführungsformen der Erfindung sowie anhand der Zeichnung. Hierbei zeigt:
- Fig. 1: die einfachste Ausführungsvariante für die Kopfeinheit einer erfindungsgemäßen Vorrichtung;
- Fig. 1a: eine beispielhafte Ausformung des Ballonrohlings;
- Fig. 1b: verschiedene Präformationen der Ballonhülle und des Trichterelementes;
- Fig. 1c: eine Zwei- oder Mehrkomponentenausführung des Trichterelementes;
- Fig. 2: eine andere Ausführungsform der Erfindung;
- Fig. 3: eine wiederum abgewandelte Ausführungsform der Erfindung mit einer Kombination aus Ankerballon und einem aus der Ankerballonhülle hervorgehenden, trans-analen Segment wie in Fig. 1 dargestellt, jedoch mit dem in Fig. 2 beschriebenen stabilisierenden Rohrstück;
- Fig. 4: eine abgewandelte Ausführungsform der Erfindung, deren transanales Segment über zwei konzentrische Schlauchlagen verfügt;
- Fig. 5: eine abermals abgewandelte Ausführungsform der Erfindung, welche die Ausführungsform nach Fig. 4 mit dem in Fig. 3 wiedergegebenen, elastisch verformbaren, sich selbst auf- und ausrichtenden Rohrgeflecht kombiniert;
- Fig. 6a: eine einfache Ausführungsform der erfindungsgemäßen Kopfeinheit;
- Fig. 6b: die Integration eines zum intra-rektalen Segment führenden Füllschlauchs;
- Fig. 6c: die Zuführung eines Spülschlauchs zum Trichterelement;
- Fig. 7: das Prinzip der Rückstülpung;
- Fig. 8: weitere Ausführungsformen des trans-analen Segments;
- Fig. 9: eine andere Ausführungsform der Kopfeinheit;
- Fig. 10: Möglichkeiten zur Kompartimentierung von Schlauchelementen der Vorrichtung;
- Fig. 11a: eine Ausführungsform der Erfindung, bei welcher ein zweigekammerter Ballon auf einem einzigen durchgehenden Trägerkörper aufgebracht ist;
- Fig. 11b: eine weitere Ausführungsform der Erfindung, bei welcher ein zweigekammerter Ballon auf einem einzigen durchgehenden Trägerkörper aufgebracht ist;
- Fig. 12a: eine Ausführungsform der Erfindung, bei welcher ein ein-gekammerter Ballon auf einem einzigen durchgehenden Trägerkörper aufgebracht ist;
- Fig. 12b: eine weitere Ausführungsform der Erfindung, bei welcher ein eingekammerter Ballon auf einem einzigen durchgehenden Trägerkörper aufgebracht ist;
- Fig. 13a: die erfindungsgemäße Gesamtvorrichtung mit allen Komponenten;
- Fig. 13b: die Anordnung aus Fig. 13a, wobei der Auffangbeutel aufgeschnitten wiedergegeben ist;
- Fig. 14: eine bevorzugte Ausführungsform eines Auffangbeutels, kombiniert mit einer Spüleinrichtung; sowie
- Fig. 15: eine Vorrichtung zur Entwicklung einer treibenden Kraft für einen Einlauf.

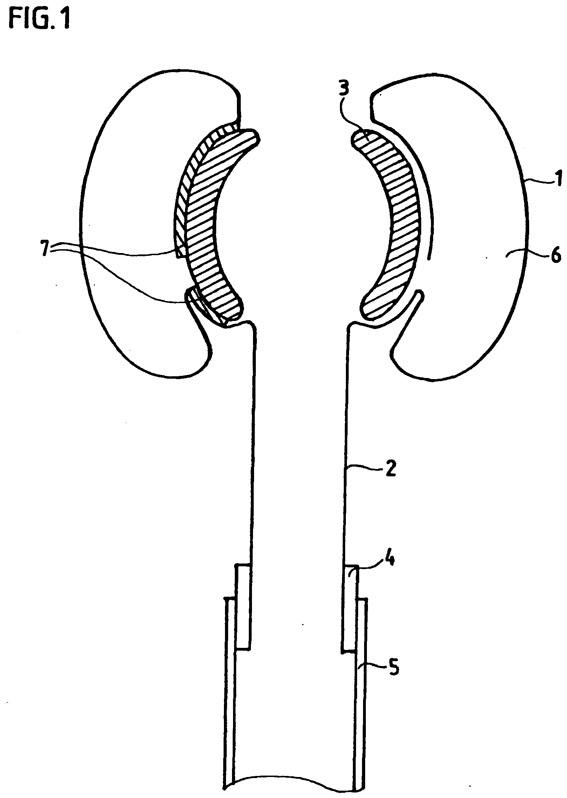

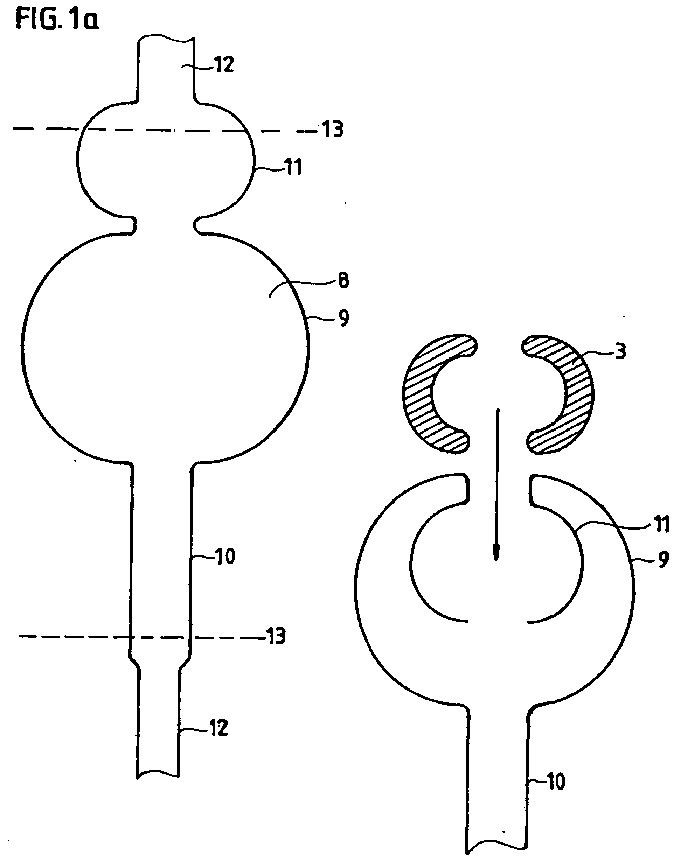

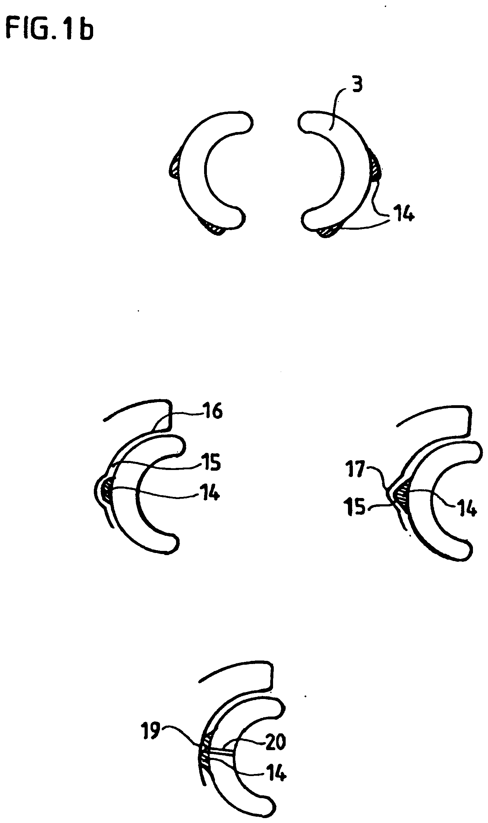

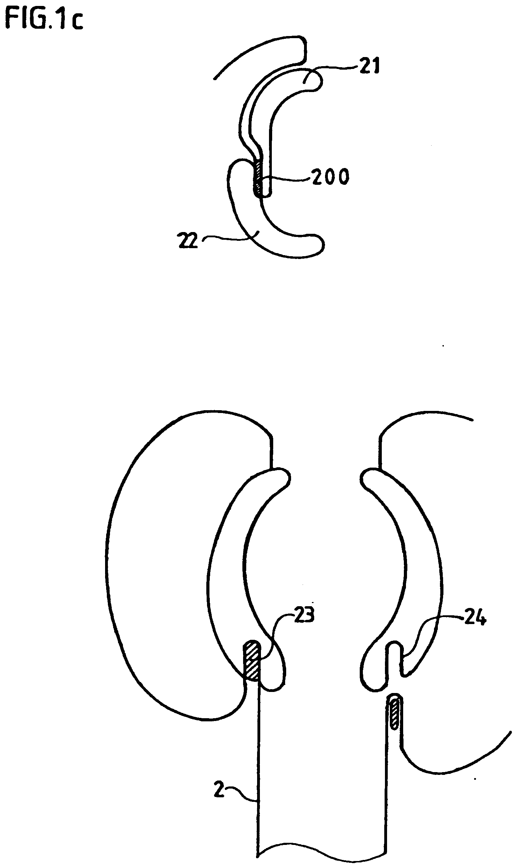

Intra-rektaler Ankerballon 1 und trans-anales Zwischensegment 2 werden aus einem einzigen gemeinsamen Schlauch-Rohling 8 gefertigt, wobei das transanale Segment 2 der ausgeformten Ballonfolie 8 lediglich eine ein-lagige Wandung aufweist. Intra-rektales und transanales Segment 1, 2 der Ballonfolie 8 bilden in der dargestellten Vorrichtungsvariante zwar eine strukturelle Einheit, weisen im montierten und auf/an einem Trichter fixierten Zustand jedoch eine funktionelle Trennung auf. Wie Fig. 1 zeigt, ist der Schlauch-Rohling 8 im Bereich des intra-rektalen Ankerballons 1 in sich zurück umgestülpt, insbesondere nach innen bzw. derart, dass ein ggf. verbleibender Spalt zwischen der Endkante des umgestülpten Schlauchs 8 und einem mittleren, der Endkante zugewandten Schlauchabschnitt 8 an der Innenseite des intra-rektalen Ankerballons 1 liegt; infolge der Umstülpung hat der intrarektale Ankerballon 1 im Wesentlichen eine torusförmige Struktur.

Beide Segmente 1, 2 der Folie 8 werden durch ein von distal her in die innere, zentrale Ausnehmung des Ankerballons 1 eingesetztes, etwa rotationssymmetrisches Element, das sog. Trichterelement 3, voneinander separiert bzw. dichtend voneinander getrennt. Der Körper des Trichterelements 3 deckt einen ggf. zwischen der Endkante des umgestülpten Schlauchs 8 und einem mittleren, der Endkante zugewandten Schlauchabschnitt 8 an der Innenseite des intra-rektalen Ankerballons 1 verbleibenden Spalt ab. Durch eine dauerhafte, feste Verbindung der Ankerballonhülle 1 mit dem Trichterelement 3 entsteht ein selbst im Bereich eines Spaltes der Ballonhülle 8 verbleibenden Spaltes abgeschlossenes bzw. abgedichtetes und daher mit einem Fluid befüllbares Kompartiment 6, welches sich nach Art einer Manschette um die seitlichen bzw. radial außen liegenden Partien des Trichterelements 3 herum erstreckt bzw. bei der Befüllung entfaltet. Die Verbindung kann durch besonders gestaltete, und im folgenden näher beschriebene Präformationen 7 der Trichteroberfläche einerseits und/oder der Ballonfolie andererseits vereinfacht bzw. verbessert werden.

Dem trans-analen Segment 2 schließt sich nach proximal hin ein die Kopfeinheit abschließendes Konnektionselement 4 an, welches den Übergang zum Drainageschlauch 5 bildet.

Der ausgeformte Ballonrohling 8 weist einen mittelständigen, nach außen etwa sphärisch ausgeformten Bereich 9 auf, der im montierten Zustand die radial außen liegende Hülle des intra-rektalen Ankerballons 1 bildet. Ein proximal der Sphäre 9 sich erstreckender Fortsatz 10 hat eine zylindrische Gestalt und entspricht im montierten Produkt dem trans-analen Abschnitt 2 der Kopfeinheit. Ein entgegengesetzt gerichteter Fortsatz als Trichterloge 11 ausgebildet zur Aufnahme des Trichterelements 3, ähnlich einer durch Faszien gebildeten Umgreifung einer Muskelgruppe im Rahmen einer Muskelloge. Jeweils endständig schließen sich Anteile des beim blow-molding eingesetzten Schlauchrohlings 12 an. Der Ballonrohling kann zur Herstellung der in Fig. 1 dargestellten Kopfeinheit beispielsweise in den Schnittebenen 13 abgesetzt werden, wie aus Fig. 1a zu entnehmen ist.

Die Fig. 1a zeigt in der rechten Darstellung ferner, wie ein die Trichterloge 11 bildender Bereich des Ballonrohlings bei der Montage in das Innere der intrarektalen Sphäre 9 zurückgestülpt wird, und wie in einem anschließenden Schritt das Trichterelement 3 in den nun invertierten Logenbereich 11 eingesetzt bzw. eingesteckt werden kann.

Die innere Kontur des invertierten Logenbereichs 11 entspricht dabei vorzugsweise passgenau der Außenkontur des in die intra-rektale Balloneinheit 1 eingesetzten Trichterelementes 3. Die Loge 11 kann sowohl für eine nahezu vollständig den Umfang umfassende, als auch partielle Aufnahme des Trichterelements 3 ausgeformt bzw. bemessen sein.

Fig. 1b zeigt verschiedene Präformationen der Ballonhülle 8 und des Trichterelementes 3, die jeweils bzw. vor allem in Kombination die komfortable Montage, die torsionsfreie längsaxiale Ausrichtung und dauerhafte dichtende Verbindung von Ballonfolie 8 und Trichterelement 3 erleichtern:
Jene Anteile des intra-rektalen und/oder trans-analen Ballonsegmentes 1, 2, welche dem Trichterelement 3 im endmontierten Zustand dauerhaft flächig anliegen, werden vorzugsweise geringgradig kleiner bemessen als die entsprechenden, dem Ballon 8 anliegenden Strukturen des Trichterelements 3. So kann gewährleistet werden, dass sich die Ballonhülle 8 im montierten Zustand allseitig unter leichter Dehnung faltungsfrei an das Trichterelement 3 schmiegt. Trichterelement 3 und Loge 11 werden geometrisch bevorzugt so gestaltet, dass der Trichter 3 beim Einsetzen in die Loge 11 quasi in seiner Montageposition einrastet. Die Außenkontur des Trichters 3 kann zu diesem Zweck mit einer geeigneten, dreidimensionalen Grundform wie z.B. der einer Olive, einer Sphäre oder einer Hantel versehen sein; die Innenkontur der Loge 11 ist als Pendant dazu gestaltet. Bevorzugt ist die Außenkontur des Trichterelements 3 zumindest in einem Bereich doppelt konvex gewölbt, d.h., sowohl in axialer Richtung als auch in azimutaler Richtung, vorzugsweise ist sie vollständig doppelt konvex gewölbt, d.h., im gesamten Bereich der Außenkontur. Damit korrespondiert eine Innenkontur der Trichterloge 11, die zumindest bereichsweise doppelt konkav gewölbt ist, d.h., sowohl in axialer Richtung als auch in azimutaler Richtung; vorzugsweise ist der gesamte, an dem Trichterelement 3 anliegende Bereich doppelt konkav gewölbt.

Zur weiteren Erleichterung der Montage dünnwandiger Ballonfolien 8 kann die Oberfläche des Trichterelementes 3 mit besonderen, zirkulär um die Trichteroberfläche verlaufenden, ringartigen Erhebungen 14 versehen sein, die im Kontaktbereich mit der Ballonhülle 8 deren Auflagespannung erhöhen und so quasi dichtend wirken. Derartige, dichtende oder begrenzende Strukturen sind besonders dann von Vorteil, wenn die Ballonhülle 8 im prall befüllten Zustand auf einer Schaftgrundlage aufgebracht ist, dort in Montageposition bewegt, und über die Längsachse hinweg entwunden und ausgerichtet wird. Auch die gezielte Einbringung von Klebstoff oder Lösungsmittel zwischen einem Schaft- und/oder Trichterelement 3 einerseits und der Ballonhülle 8 andererseits, insbesondere in einem begrenzten und/oder definierten Bereich, kann dadurch verbessert werden. Beispielsweise kann so in einen hinter einer Erhebung 14 gelegenen Spalt 15 zwischen dem Trichter 3 einerseits und einem am Trichter 3 anliegenden Bereich 16 der Ballonhülle 8 andererseits räumlich begrenzt ein Klebemittel eingebracht, insbesondere eingespritzt werden, ohne dass diese Klebemittel in den Bereich jenseits der ringförmigen Erhebung 14 verlaufen kann.

Um die Positionierung und vor allem die axiale Ausrichtung und Fixierung der Hülle noch weiter zu erleichtern, kann die ringförmig bzw. zirkulär um den Umfang der Oberfläche des Trichterelements 3 verlaufende konvexe Erhebung 14 durch eine korrespondierende, zu der Erhebung 14 etwa komplementäre, konkave Präformierung 17 der anliegenden Ballonhülle verbessert werden. Es wird so ein weiterer Rastungseffekt bei der Positionierung der Folie auf dem Trichter ermöglicht. Die Hülle 8, 16 wird in ihrem Lauf quasi geschient, ohne die präzise Positionierung auf der Unterlage, bzw. der Erhebung 14, zu verlassen, und kann komfortabel über die Ballonlängsachse entwunden und über die Längsachse ausgerichtet werden.

Es kann desweiteren zwischen dem Trichterelement 3 und der Ballonfolie 8, 16, vorzugsweise im Bereich von deren Präformationen, insbesondere im apikalen Kontaktbereich der Präformationen, ein Zwischenraum 18 vorgesehen sein, der ein die Strukturen dauerhaft verbindendes Medium, z.B. Klebstoff, aufnimmt. Die kontrollierte Verteilung eines Klebemediums zwischen Ballonhülle und Trichteroberfläche kann in entsprechendem Sinne durch eine apikal auf der ringförmigen Erhebung 14 verlaufende Nut 19 erfolgen. Das verklebende Medium kann dabei über eine kanalartige Präformation 20 vom Trichterlumen her zugeleitet und eingebracht werden.

Die Erfindung sieht weiterhin vor, dass das Trichterelement 3 und die Ballonfolie 8, 16 durch Verschweißung miteinander verbunden werden, vorzugsweise im Bereich wenigstens einer ringförmig und/oder zirkulär um den Trichterumfang verlaufenden, konvexe Präformation. Dies ist - bedingt durch die so lokal erreichte Steigerung der Auflagespannung der Folie 8 auf der Unterlage - vorteilhaft für die Verschweißung der beiden Strukturen, denn ein entsprechendes thermisches Schweißwerkzeug kann dort aufsetzen und eine verlässlich faltungsfreie Verbindung der Oberflächen herstellen.

Wie Fig. 1c zeigt, kann das Trichterelement 3 beispielsweise aus zwei ineinander steckbaren oder auf entsprechende Weise verbindbaren Teilen bestehen, bspw. aus ringförmigen Teilen 21, 22, welche in axialer Richtung hintereinander angeordnet sind. Bei der Montage werden die jeweiligen Teile 21, 22 vorzugsweise separat mit dem distalen bzw. proximalen Ballonende dauerhaft verbunden, z.B. durch Klebung. Sind die Trichterkomponenten 21, 22 - zumindest bereichsweise - mit der Ballonhülle 8 dauerhaft verbunden, können sie anschließend über die Längsachse der Ballonhülle 8 entwunden werden, und so, axial ausgerichtet, ohne Torsion der Ballonhülle 8, permanent dichtend zusammengefügt werden.

Die Verbindung von Ballonhülle 8 und Trichterkomponenten 21, 22 kann auch derart erfolgen, dass die Verbindungsstellen im Bereich der Fügung der Trichterkomponenten zu liegen kommen.

Beispielsweise kann das distale Ballonende mit der Trichterkomponente 21 verklebt sein, insbesondere in dem Fügungsbereich 200 mit der Komponente 22. Der Logenanteil 11 der Ballonhülle 8 wird dabei vorzugsweise am proximalen Rand der Komponente 21 aufgeklebt bzw. dauerhaft gefügt. Beide Strukturen werden dann gemeinsam in eine kongruente aufnehmende Struktur am distalen Rand der Komponente 22 eingesetzt und dort, nach axialer Ausrichtung, ebenfalls verklebt.

In ähnlicher Mehrkomponentenbauweise kann der proximale dichtende Abschluss der Ballonhülle 8 mit dem Trichterelement 3 erreicht werden. Diese Montagetechnik beruht auf einem separaten, ringförmigen Element 23. Der Ring 23 kann beispielsweise von proximal her über das trans-anale Segment 2 bis zur proximalen Fixierungsposition der Ballonhülle 8 auf dem Trichter 3 geschoben werden, insbesondere bis zum Übergang vom intra-rektalen Ballon 1 zum trans-analen Segment 2, und dort optional durch Klebung, Verschweißung od. dgl. an dem transanalen Segment 2 fixiert werden. Eine rastende Platzierung des Rings 23 kann durch eine dem Ring 23 entsprechende Präformierung am proximalen Ende der Ballonhülle 1 erreicht werden.

Der Ring 23 wird dann, unter Mitnahme des an ihm fixierten distalen Endes des trans-analen Segments 2, in eine entsprechend ausgeformte, ringnutartige Präformierung 24 am proximalen Ende des Trichterelementes 3 eingesteckt und kann dort beispielsweise durch Verklebung, Verschweißung od. dgl. fixiert werden.

Das Ringelement 23 kann eine geringfügig höhere Shore-Härte aufweisen als die restlichen Trichterkomponenten 21, 22, um so, bei leicht vergrößertem oder verringertem Durchmesser im Vergleich zur aufnehmenden Präformierung, eine Verrastung der beiden Trichterkomponenten 21, 22 unter leichter Spannung zu ermöglichen.

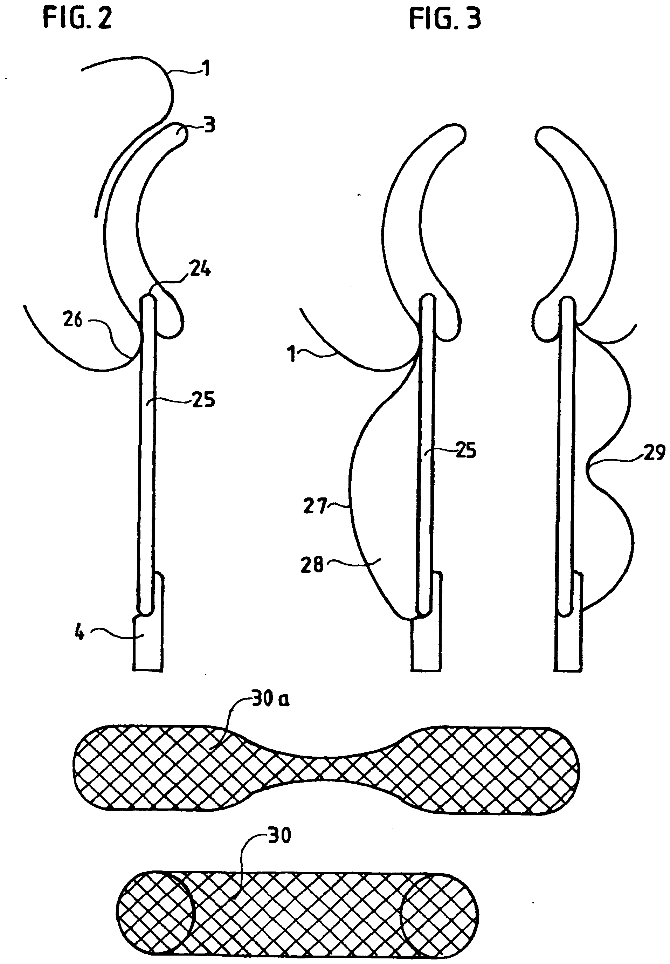

Die Ausführungsform der Vorrichtung in Fig. 2 verfügt anstatt eines aus dem Ankerballon 1 hervorgehenden, nach proximal verlängerten Ballonendes, wie z.B. in Fig. 1 dargestellt, über ein separates, trans-anal verlaufendes dünnwandiges Rohr- bzw. Schlauchstück, welches den Trichter 3 mit dem prä-analen Konnektor 4 durchgängig verbindet. Hierbei handelt es sich um ein Element aus einem elastischen Werkstoff mit hoher Rückstellkraft, welches sich, trotz möglichst dünnwandiger Ausführung bei radialer Verformung bzw. bei Torsion über die Längsachse selbständig aufrichtet bzw. entwindet und so spontan, in seinen spannungsarmen, lumen-offenen Ausgangszustand zurückkehrt.

Die Abbildung zeigt beispielhaft, wie die Verbindung zwischen dem Rohr- und/oder Schlauchstück 25 und dem Trichterelement 3 durch eine präformierte Ringnut 24 am proximalen Trichterende 26 gestaltet werden kann. Das proximale Ende 26 der Ballonhülle 1 kann dabei über das distale Ende des Elementes 25 gelegt, gestülpt oder in sonstiger Weise geführt werden, dass dieses durch das Einstecken des distalen Endes des trans-analen Segmentes in die Ringnut 24 dauerhaft dichtend mit dem Trichterelement 3 verbunden wird.

Fig. 3 zeigt eine Kombination von Ankerballon 1 mit einem aus der Ankerballonhülle 8 hervorgehenden, trans-analen Segment 2 wie in Fig. 1 dargestellt, mit dem in Fig. 2 beschriebenen stabilisierenden Rohr- und/oder Schlauchstück 25. Das trans-anale Segment bildet dabei ein separat befüllbares Kompartiment 28 aus, welches zwischen dem innen liegenden Rohr- und/oder Schlauchstück 25 und der äußeren, nach proximal verlängerten Anker-Ballonhülle 27 entsteht. Bei Befüllung dieses Kompartiments 28 kann, abhängig von der Volumendehnbarkeit der Hülle bzw. ihres bei der Herstellung ausgeformten Durchmessers, durch radiale Ausdehnung der äußeren Hülle 27 die Dichtung zum Anus hin verbessert werden. Bei forcierter Befüllung kann darüber hinaus das Drainagelumen der Kopfeinheit zum Zentrum hin verengt und annähernd verschlossen werden.

Alternativ zur Ausformung der Hülle 27 aus dem proximalen Ausläufer des Ankerballons 1 kann als Hülle 27 auch ein separat hergestellter Ballon- oder Folienschlauchkörper zur Anwendung kommen. Die das Kompartiment 28 nach außen hin begrenzende Hülle 27 kann darüber hinaus hantel- oder sanduhrförmig präformiert sein. Die bevorzugt mittige Taillierung 29 ist dabei derart ausgeformt, dass sie die Strukturen des Analkanals aufnimmt. Wird das trans-anale Kompartiment 28 mit Druck beaufschlagt, wie dies z.B. bei der Instillation der Irrigationsflüssigkeit erfolgt, gewährleistet die beschriebene Präformation 29 zum einen eine sichere Verankerung des Kopfteils der Vorrichtung, zum anderen trägt sie zur Dichtung gegen die von rektal her anlastende Spülflüssigkeit bei.

Zur Fixierung und zum dichtenden Abschluss der für diese Ausführungsform erforderlichen Komponenten im Übergangsbereich zwischen intra-rektalem und trans-analem Kompartiment 6, 28 können die zuvor beschriebenen Techniken - bspw. Verklebung, Verschweißung, ggf. im Bereich einer Präformierung, etc. - verwendet werden.

Analog kann das bevorzugt aus dem Ankerballon 1 hervorgehende Folienschlauchsegment 27 mit einem radial verformbaren, sich den transanalen Konturen unter leichter Spannung anschmiegenden Rohrgeflecht 30 kombiniert werden. Das Rohrgeflecht 30 weist im nichtverformten, spannungsarmen Ausgangszustand ein offenes Lumen von ca. 2-3 cm auf. Die sich bei Lumen-Einengung entwickelnde, radial gerichtete Rückstellkraft auf den Anus ist dabei gering und schließt die Entstehung von Druckulcera aus. Kommt es beim Defäkationsreflex zu einer Tonusreduktion des analen Schließmuskels, folgt das Rohrgeflecht dem sich dann öffnenden trans-analen Kanal und erleichtert so den Stuhlabfluß. Das Rohrgeflecht 30 verbindet den intra-rektalen Trichter 3 mit dem prä-analen Konnektor 4 vorzugsweise durchgehend. Es ist bevorzugt im Inneren des Schlauchsegmentes 8 angeordnet, kann jedoch alternativ auch über dessen äußere Oberfläche verlaufen. Im radial eingeengten Zustand 30a weist das vorzugsweise aus starren, biegbar verformbaren Filamenten aufgebaute Geflecht 30 einen deutlichen Längenzuwachs auf, der durch eine entsprechende Längenvorgabe des umgebenden trans-analen Folienschlauches 27 berücksichtigt ist.

Die Montage der anteiligen Komponenten folgt den zuvor beschriebenen Techniken.

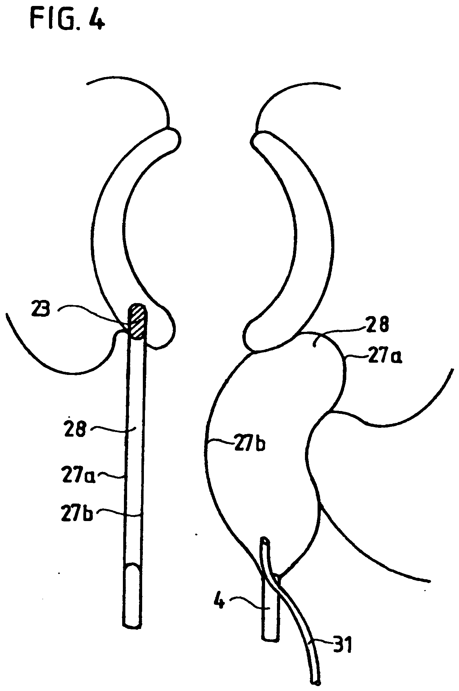

Fig. 4 stellt eine Ausführungsform dar, deren trans-anales Segment über zwei konzentrische Schlauchlagen 27a und 27b verfügt. Diese können, wie zuvor beschrieben, aus den verlängerten Enden des intra-rektalen Ballons 1 bestehen. In der bevorzugten Ausführungsform geht die innere Schlauchlage 27b aus der proximalen Verlängerung der intra-rektalen Ballonsphäre 9 hervor. Die äußere Schlauchlage 27a wird bevorzugt als separates dünnwandiges, zylindrisch oder auch dem Analkanal kongruent präformiert ausgeformtes Folienschlauchelement gestaltet.

Alternativ können die beiden Lagen auch aus jeweils separat gefertigten Schlauchfolien bestehen.

Der zwischen den beiden Schlauchlagen 27a, 27b entstehende Raum 28 kann über eine über den Konnektor 4 geführte Zuleitung 31 von extra-korporal mit einem Medium partiell oder vollständig befüllt oder auch vollständig evakuiert werden.

Im evakuierten Zustand (entsprechend etwa Fig. 4 linke Hälfte) liegen die beiden Schlauchlagen 27a, 27b einander fest an und verhalten sich quasi wie eine ein-lagige Wandung. Im befüllten Zustand (entsprechend etwa Fig. 4 rechte Hälfte) trennen sich die beiden Lagen 27a, 27b voneinander, der Raum 28 ist maximal geöffnet.

Im prall befüllten Zustand dehnt sich die innere Lage 27b zum Zentrum des Drainagelumens hin aus und verschließt dieses flüssigkeitsdicht. Die äußere Lage 27a hingegen dehnt sich zur analen Wandung hin aus und schmiegt sich dieser, der jeweiligen Anatomie folgend, ebenfalls dichtend an.

Bei teilweiser Befüllung bzw. Befüllung mit wenigen Millilitern Füllmedium trennen sich die beiden Lagen 27a, 27b. Die beiden dünnwandigen Folien 27a, 27b gleiten, durch das Füllmedium quasi reibungsfrei gelagert, frei verschieblich zueinander. Durch das freie Spiel und Gleiten der Folienlagen 27a, 27b können im besonders sensiblen Bereich des Analkanals Läsionen, wie sie durch statisch anliegende, wenig dynamische Folien bekannt sind, besser vermieden werden.

Die Verbindung der anteiligen Komponenten bzw. Folienlagen 27a, 27b mit dem proximalen Trichterende 26 kann auf der Basis eines ringförmigen Trägers 23 erfolgen, der in eine ringförmige Präformierung 24 am proximalen Rand des Trichterelements 3 eingesetzt wird. Die innere Lage 27b kann dabei wie in Fig. 1c über den Ring 23 geführt und mit dem Trichter 3 verbunden bzw. verrastet werden. Die äußere Lage 27a kann in einem vorausgehenden Montageschritt an ihrem distalen Ende auf dem Ring 23 aufgeklebt werden.

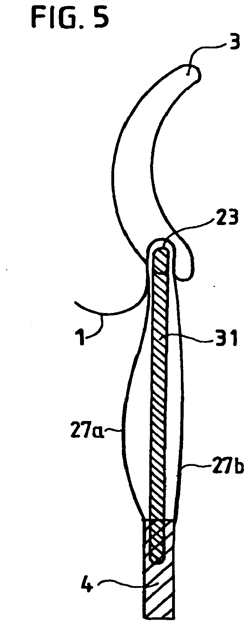

Fig. 5 kombiniert die zuvor beschriebene Ausführung mit dem in Fig. 3 beschriebenen, elastisch verformbaren, sich selbst auf- und ausrichtenden Rohrgeflecht 31. Alternativ zu einem Rohrgeflecht 31 kann das zuvor beschriebene Schlauch- oder Rohrelement 30 verwendet werden.

Die zuvor beschriebenen Elemente 30, 31 werden bevorzugt zwischen den beiden Folienlagen 27a und 27b, das Trichterelement 3 mit dem Konnektor 4 verbindend, angeordnet.

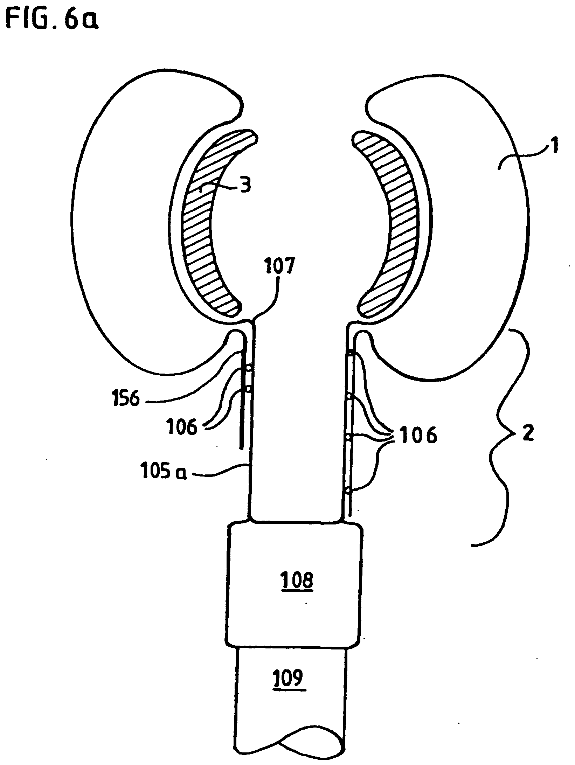

Die Fig. 6a zeigt eine andere Ausführungsform der Erfindung. Intra-rektaler Ankerballon 1 und trans-anales Zwischensegment 2 sind dabei aus einem einzigen, gemeinsamen, zuvor vollständig ausgeformten Schlauchballon-Rohling 8 gefertigt, wobei ein Ende 105a des Ballonschlauchs 8 durch dessen anderes Ende 105b hindurch gestülpt wird. Die beiden Enden 105a, 105b liegen einander im trans-analen Abschnitt 2 der Vorrichtung konzentrisch an und sind im Kontaktbereich 106 vorzugsweise durch eine flächig wirkende Verbindungstechnik miteinander dauerhaft verbunden. Der Kontaktbereich 106 zwischen den beiden Ballonenden 105a, 105b beginnt vorzugsweise am unteren Rand 107 der Trichterloge 11 bzw. wenige Millimeter nach proximal von diesem beabstandet. Er erstreckt sich über die teilweise oder volle Länge des trans-analen Segmentes 2 der Vorrichtung.

Der Kontaktbereich 106 der Schlauchenden 105a und 105b stellt sich somit doppellagig dar. Die Lagen sind zwar vorzugsweise durchgängig fest miteinander verbunden. Eine partielle Kammerung im Kontaktbereich 106 bzw. eine partiell flächige Verbindung ist denkbar. Eine Befüllmöglichkeit oder Verbindung mit einem anderen mit Druck beaufschlagten Raum eventueller Kompartimente, mit Ausnahme der Umgebung, besteht jedoch nicht, insbesondere besteht keinerlei durchgängige Verbindung zu der Kammer 6 innerhalb des intra-rektalen Ballons 1. Die vorzugsweise durchgehend doppelte Materiallage im Kontaktbereich 106 stattet das transanale Segment 2 mit höherer Steifigkeit aus und wirkt so vor allem einer axialen Torsion und damit verbundenen Einengung des Drainagelumens entgegen. Zum anderen gewährleistet sie die verbesserte Neigung zur spontanen Aufrichtung des Segmentlumens.

Intra-rektales und transanales Segment 1, 2 der Ballonfolie 8 bilden in der dargestellten Vorrichtungsvariante zwar eine strukturelle Einheit, sind funktionell jedoch voneinander entkoppelt.

Dem trans-analen Segment 2 schließt sich nach proximal hin ein Konnektionselement 108 an, welches den Übergang zum Drainageschlauch 109 bildet.

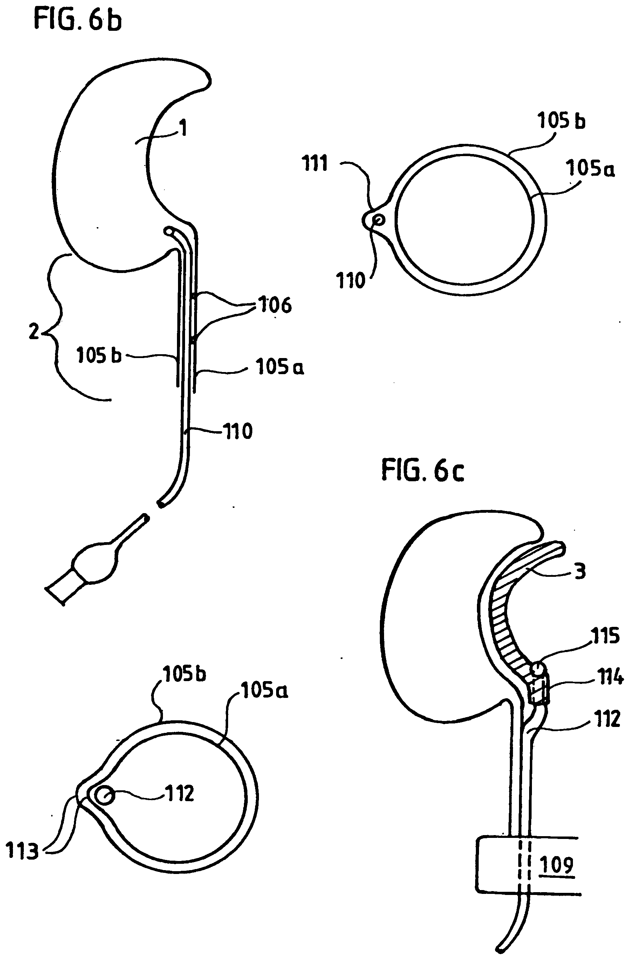

Um eine Füllung des intra-rektalen Ballons 1 zu gewährleisten, wird die Integration eines Füllschlauches 110 in den Kontaktbereich 106 der beiden Schlauchenden 105a und 105b empfohlen. Dies kann im einfachsten Fall durch Einlegen des Füllschlauchs 110 zwischen die beiden konzentrisch angeordneten Folienlagen bzw. Schlauchenden 105a, 105b erfolgen, wie in Fig. 6b dargestellt. Die Fixierung und Abdichtung kann durch Verklebung oder Verschweißung der zu verbindenden Komponenten 105a, 105b, 110 erfolgen.

Die Aufnahme des Füllschlauches 110 in die Kontaktlage 106 kann durch entsprechende, paßgenaue nutartige, den zwischengelegten Schlauch 110 aufnehmende Präformierung(en) 111 in den Schlauchenden 105a und/oder 105b erleichtert werden, wie in fig. 6b rechts zu erkennen ist.

Um die Zuleitung von Spülflüssigkeit zum intra-rektalen Segment 1 hin zu gewährleisten, wird die Integration eines Spülschlauches 112 beschrieben, der vorzugsweise im Inneren des Drainagelumens angebracht ist bzw. dort in eine entsprechende nutartige Präformierung 113 im Inneren des trans-analen Segmentes 2 fixiert wird, wie in Fig. 6c dargestellt.

Im intra-rektalen Abschnitt 1 der Vorrichtung kann der Spülschlauch 112 von einer entsprechend ausgeformten Präformierung 114 im Trichterelement 3, z.B. durch Auf- oder Einstecken auf/in diese, aufgenommen und gehalten werden. Die Präformierung 114 kann eine Mündungsöffnung 115 zum Drainagelumen ausbilden.

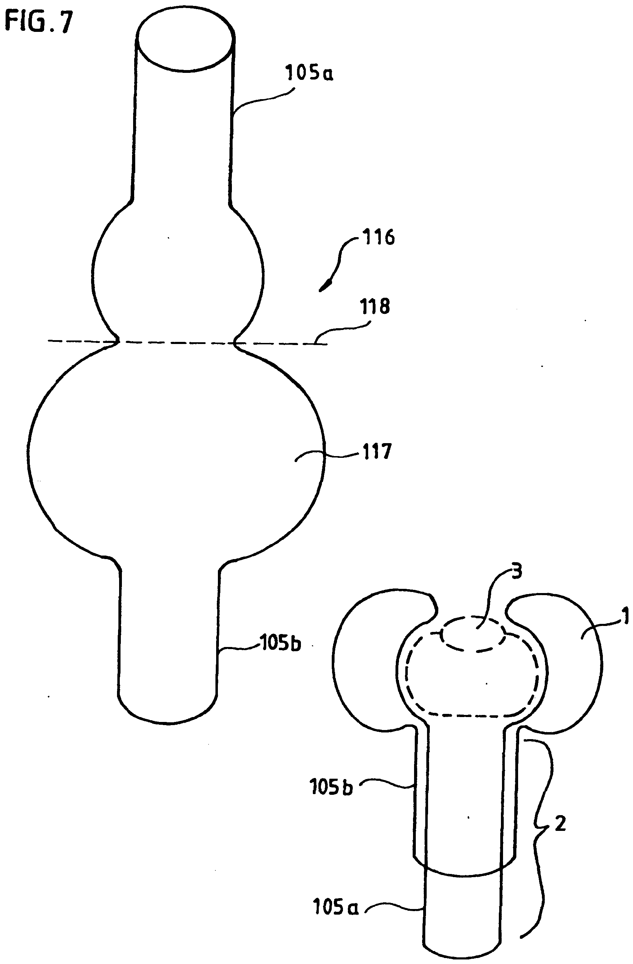

Wie Fig. 7 erkennen läßt, weist der ausgeformte Ballonrohling 116 eine mittelständige sphärische Formation 117 auf, die sich im montierten Zustand als Hülle des intra-rektalen Ankerballons 1 darstellt. Der Fortsatz 105a der Sphäre 117 hat vorzugsweise eine länglich zylindrische Gestalt und entspricht im montierten Produkt dem trans-analen Abschnitt 2 der Kopfeinheit. Bei der Montage erfolgt eine Rückstülpung des Endes 105a durch das gegenüberliegende Ende 105b in der Weise, dass sich direkt bzw. nahe am distalen, patienteninneren Ende der Vorrichtung die Linie 118 als Rückstülpungslinie einstellt.

Der Fortsatz 105a kann optional eine Trichterloge 11 ausbilden.

Die innere Kontur der invertierten Loge 11 entspricht dabei vorzugsweise passgenau der Außenkontur des in die intra-rektale Einheit eingesetzten Trichterelementes 3.

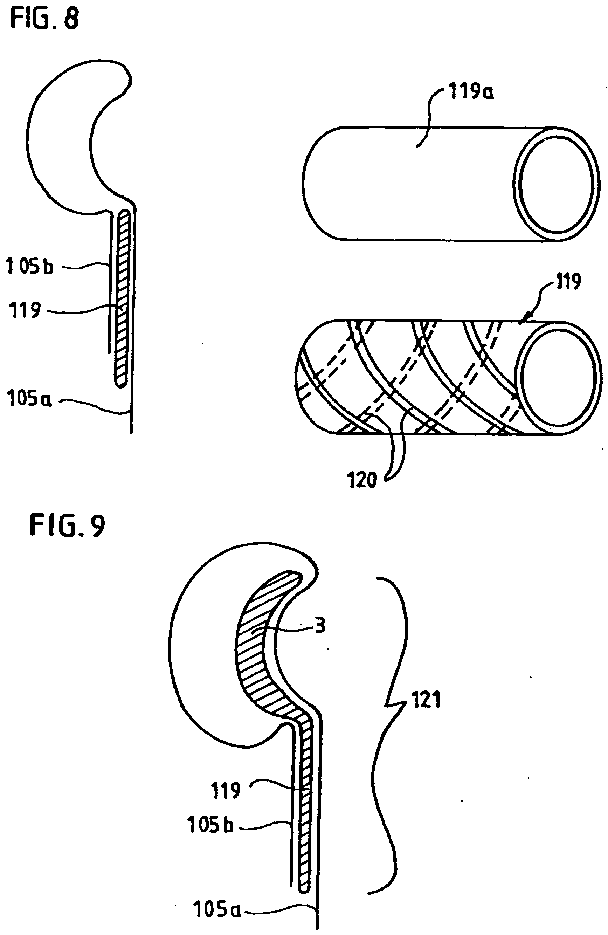

Bei der Ausführungsform gemäß Fig. 8 werden die Schlauchenden 105a und 105b nicht direkt miteinander, sondern jeweils mit den Oberflächen einer dazwischen liegenden, hülsen- oder schlauchartigen Komponente 119 verbunden, deren Zweck es ist, die mechanischen Eigenschaften der transanalen Struktur 2 gezielt zu modifizieren.

Im idealen Falle stellt sich das trans-anale Segment 2, mit oder ohne einer derartigen Ergänzung, mechanisch so dar, dass es sich ohne äußere Krafteinwirkung, also z.B. im frei entfalteten Zustand, ohne dem Analkanal anzuliegen, durch seine elastische Kraft in seinem Querschnitt kreisrund aufrichtet. Bei geringer, von außen anlastender Wirkkraft, soll es zum Kollaps bzw. einer weitgehend lumen-verschließenden Verformung des Segmentes 2 kommen.

Ferner soll durch Auswahl einer geeigneten elastischen Hülse 119 eine gewisse Verwindungssicherheit im trans-analen Segment 2 der Vorrichtung hergestellt werden. Die Elastizität der Hülse 119 soll daher so gewählt werden, dass sich das transanale Segment 2 bei erfolgter axialer Torsion auch in situ weitgehend selbständig entwindet und in seinen spannungsarmen Ausgangszustand zurückkehrt.

Die Hülse 119 besteht vorzugsweise aus Polyurethan mit einem Härtegrad zwischen 30 bis 70A, welches durch Extrusion, Spritzguss, Gießen oder Tauchen hergestellt bzw. verarbeitet werden kann. Alternativ kann beispielsweise auch Silikon oder ein Material mit entsprechenden mechanischen, bzw. in erster Linie elastischen Eigenschaften verwendet werden.

Die Hülse 119 kann entweder durchgehend gleichförmig wandstark aufgebaut sein, wie in Fig. 8 rechts oben als Hülse 119a dargestellt, oder beispielsweise auch ein-läufige oder auch mehr-läufige, ggf. gegenläufige wendelartige Verstrebungen oder Versteifungen 120 aufweisen, wie in Fig. 8 rechts unten dargestellt. Die zwischen den Verstrebungen oder Versteifungen 120 liegende Fläche kann als dünnwandige Struktur mit einer Stärke von beispielsweise 0,2 bis 0,4 mm ausgeformt sein, oder bei gegenläufigem Verlauf mehrerer spiral- oder wendelförmig verlaufender Verstrebungen 120 auch fensterartig durchbrochen sein. Die vorzugsweise wendelartig verlaufenden Streben 120 können einen Durchmesser von beispielsweise 0,75 bis2,0 mm aufweisen.

Ferner kann die Hülse 119 auch aus einem elastischen Weichschaum bestehen, beispielsweise aus PUR-Schaum.

Alternativ kann die Hülse 119 auch als elastischer doppelwandiger Zylinder aufgebaut sein, wobei der Raum zwischen den Wandungen mit einem kompressiblen oder nicht- kompressiblen Medium prall gefüllt wird.

Die Hülse 119 kann mit den Enden 105a und 105b flächig, insbesondere ganz- oder großflächig oder auch nur partiell, verbunden sein. Ihre Länge entspricht mindestens der Länge des Analkanals, also ca. 3 bis 5 cm, reicht aber bevorzugt vom Trichterlement 3 bis zum Konnektor 4.

Die Hülse 119 liegt bevorzugt zwischen den Enden 105a und 105b, kann alternativ aber auch außen auf die äußere Lage 105b oder innen auf die innere Lage 105a aufgebracht sein.

Erfolgt die Anbringung auf der Innenseite des trans-analen Segmentes 2, also auf der Innenseite der Lage 105a, kann die Hülse 119 als weitere Ausführungsform auch direkt, quasi in proximaler Verlängerung, aus dem Trichterelement 3 hervorgehen bzw. mit diesem durchgängig und dauerhaft verbunden sein, wie in Fig. 9 zu erkennen.

Die Hülse 119 weist vorzugsweise bereits entsprechende Präformierungen zur Aufnahme von Belüftungs- und Spülzuleitungen auf.

Bei einer Fixierung der Hülse 119 zwischen den Schlauchenden 105a und 105b wäre auch eine Ausführung denkbar, bei der sich Trichterelement 3 und Hülse 119 als Einheit 121 darstellen. Bei der Montage dieser Ausführungsform wird die Hülse 119 auf dem Schlauchende 105a bis zur Rückfaltungslinie 118 geschoben und mit diesem flächig dauerhaft verbunden. Dann wird das Ende 105b quasi über die Einheit aus Hülse 119 und innerem Schlauchende 105a zurückgerollt. Das Ende 105b wird dann auf der Oberfläche des trans-analen Hülsensegmentes 2 festgelegt.

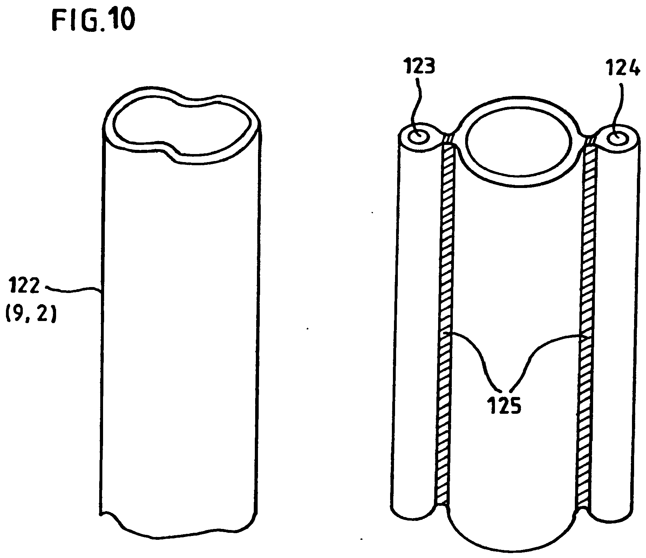

Fig. 10 zeigt weitere Möglichkeiten zur Gestaltung des transanalen Segmentes 2 sowie auch des stuhl-fortleitenden Segmentes 109 auf.

Dabei werden zuleitende Schlauchkompartimente, wie sie beispielsweise für die Befüllung des intra-rektalen Ballons 1 erforderlich sind oder auch für die Zuleitung von Spülflüssigkeit zum intra-rektalen Ende benötigt werden, durch längsgerichtete, an dem Rohschlauch 122 des transanalen und/oder stuhl-fortleitenden Segments 109 angebrachte Schweißnähte 125 quasi vom Rohschlauch 122 abgetrennt oder kompartimentiert. Die dadurch entstehenden Kompartimente 123, 124 erhalten so die Form je einer Leitung.

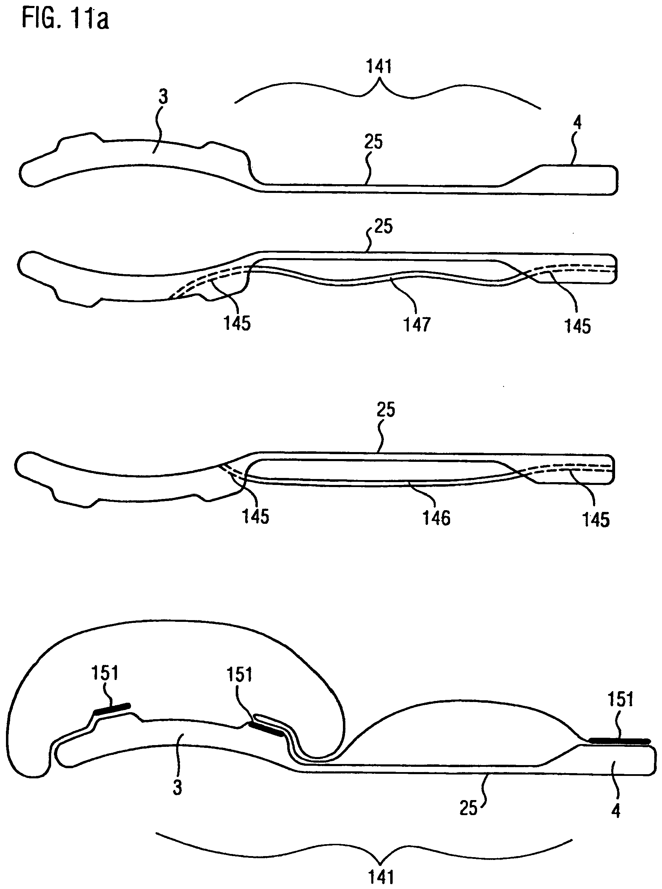

Fig. 11a beschreibt eine Ausführungsform der Erfindung, bei der das Trichterlelement 3, das trans-anale Versteifungselement 25 sowie das Konnektorelement 4 aus einer vorzugsweise durchgängigen hergestellten Einheit 141 bestehen.

Alle drei Segmente 3, 4, 25 der Einheit 141 werden vorzugsweise in einem gemeinsamen Arbeitsgang aus einem elastischen Kunststoffmaterial gefertigt. Es kommen hierzu beispielsweise Spritzguss-, Heissguss-, Tauch-, Druck- oder Szinterverfahren in Frage. Die trichterartige Olive 3 sowie der Konnektor 4 werden in ihrem proximalen Anteil vorzugsweise bereits bei der Formung im Werkzeug mit Durchstichen 145 versehen, welche die montagefreundliche Durchführung oder den Anschluss zuleitender Schlauchverbindungen, wie z.B. einer Spülleitung 146 oder einer Befüllleitung 147 des intra-rektalen Kompartiments 6 erlaubt.

Die Einheit 141 wird beispielsweise aus Polyurathan mit einer Härte zwischen 80A bis 60D gefertigt, und dabei vorzugsweise in einer durchgängigen Materialwandstärke von 0,2 bis 0,4 mm ausgeführt. Bei Verwendungen von Polyurethanen der Härtegrade 30A bis 70A, kann die Wandungsstärke auf bis zu 1,0 mm angehoben werden.

Orientierend für die funktionsrelevante Abstimmung von Wandstärke und Materialhärte ist die Selbstaufrichtung des Segmentes 141 bei von außen einwirkender verformender Kraft. Im idealen Fall stellt sich das innere Lumen des Segments 141 ohne äußere Krafteinwirkung im Querschnitt kreisrund dar. Bei einer Kraftwirkung äquivalent zu einer, über eine zentrale Strecke von 3 cm des Segmentes gleichförmig und allseitig anlastenden Wassersäule mit einer Höhe von 15 bis 25 cm, bevorzugt jedoch von 5 bis 15 cm Höhe, kommt es zum lumen-verschließenden Kollaps der Segmentwandung, wobei gegenüberliegende Wandungsanteile mindestens in direkten partiellen Kontakt kommen, sprich, sich berühren sollten.

Ebenfalls soll eine spontane Ausrichtung in den Grundzustand bei längsaxialer Torsion des Segmentes 141 erreicht werden bzw. die spontane Ausrichtung bei trans-analer Lage unterstützt werden.

Die Fig. 11a unten verdeutlicht noch einmal die Befestigungspunkte 151 des zweigekammerten Ballonkörpers 1, 2 auf dem als durchgehender Träger dienenden Segment 141, und deutet die sich bei entsprechender Fixierung entwickelnde Ballonform bei Befüllung der so geschaffenen Kompartimente 6, 28 an.

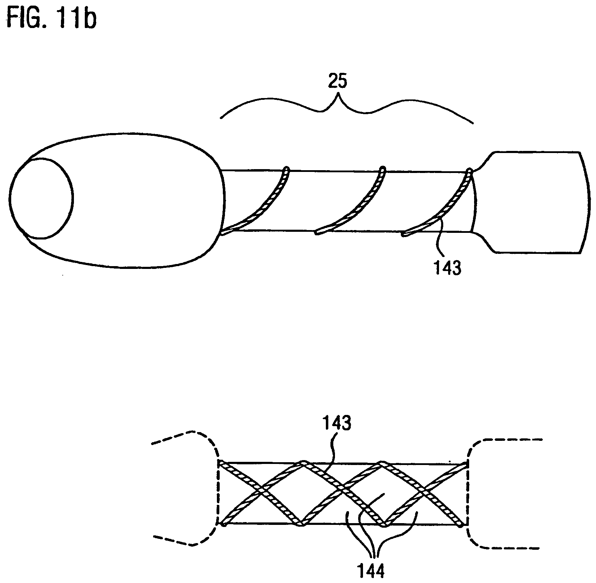

Fig. 11b zeigt eine Ausführungsform mit einer lumen-verschließenden Verformbarkeit des Versteifungs-Segmentes 125 durch wendelartige, über die innere oder äußere Oberfläche des Segmentes 125 verlaufende Streben 143, welche in einem gewissen Winkel versetzt, mehrfach in gleicher Richtung parallel zueinander angeordnet sein können. Die Streben 143 können ebenfalls einfach oder mehrfach (versetzt) gegenläufig angebracht sein. Das entstehende maschenartige Gebilde kann in seinen zwischen den Streben angeordneten Bereichen 144 dünnwändige Flächen aufweisen, die auf Wandstärken bis zu 0,1 mm hin reduziert werden können.

Die Streben 143 dienen bei Gießverfahren ferner der Gewährleistung eines ausreichend schnellen Flusses der Kunststoffmasse in der Gussform, wenn zuvor die beschriebenen, sehr dünnwändigen Flächen 144 erfolgreich ausgeformt werden sollen.

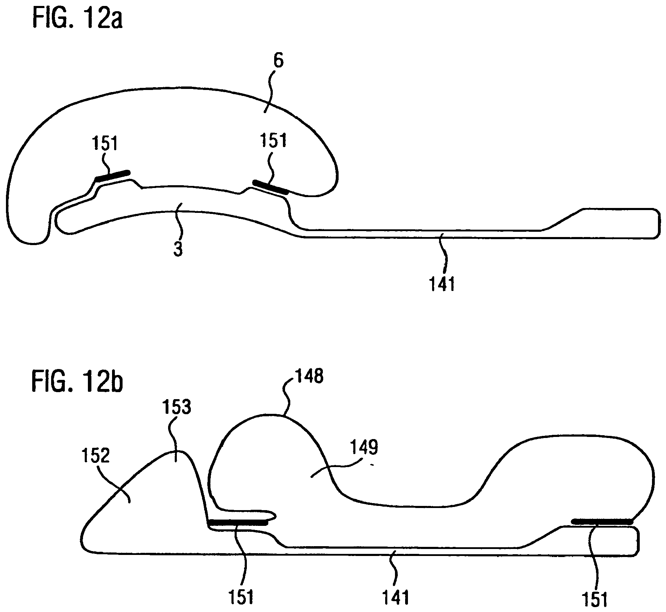

Fig. 12a zeigt den zuvor beschriebenen durchgehenden Grundkörper 141 kombiniert mit einem Ballonelement 1, welches lediglich eine einzige befüllbare intra-rektale Kammer 6 ausbildet. Der Ballon 1 sitzt an den Befestigungspunkten 151, welche zirkulär um den Umfang der Trichtersektion 3 verlaufen. Ein zweites trans-anales Ballonsegment ist hierbei nicht vorgesehen.

Fig. 12b zeigt eine Ausführungsform mit einem Segment 141, auf das lediglich eine trans-anale Ballonkomponente 148 mit einem Kompartiment 149 aufgebracht ist, während eine intra-rektale Ballonkomponente jedoch fehlt.

Der trans-anale Ballon 148 kann vorzugsweise sanduhrförmig ausgeführt sein, um sich den Konturen des Analkanals im Sinne einer besseren Verankerung in situ opitmal anschmiegen zu können. Entsprechend kann die ballonlose, trichterförmige Olive 152 einen entsprechend vergrößerten Durchmesser aufweisen, und eine ausreichende, äußere Auflagefläche 153 bieten, um die Vorrichtung im Rektum ausreichend gut zu verankern.

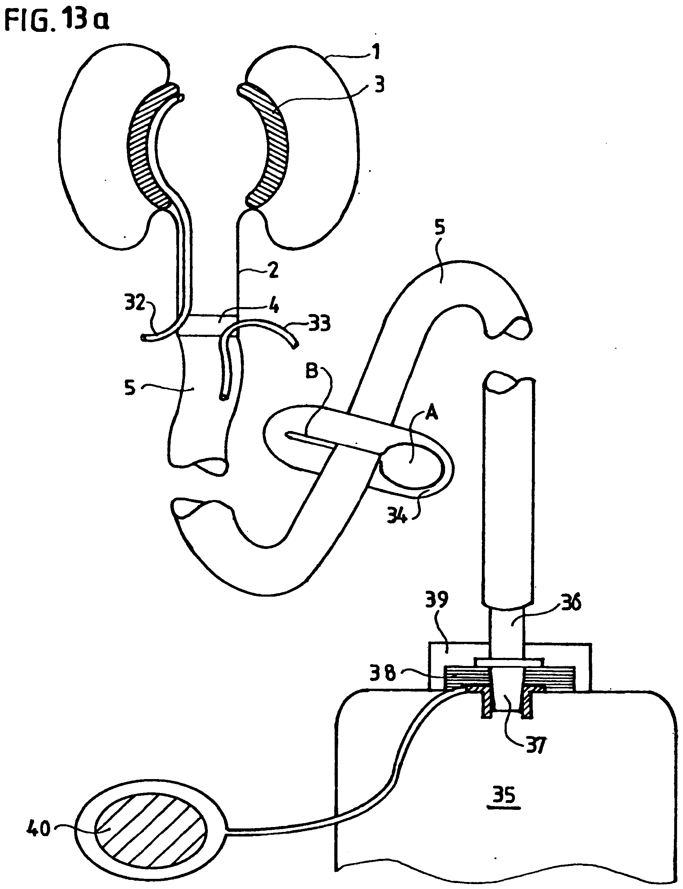

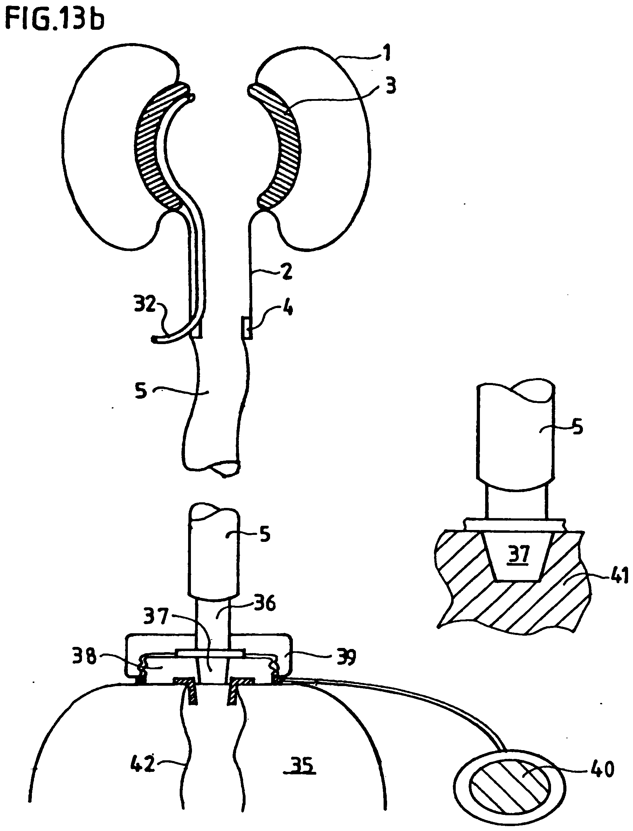

Im Folgenden werden die einzelnen Bauelemente der Vorrichtung in ihrer bevorzugten technischen Ausführung näher beschrieben. Die Gesamtvorrichtung mit allen beschriebenen anteiligen Komponenten wird in Fig. 13 dargestellt.

Der Intra-rektale Ankerballon 1 und das trans-anales Segment 2 werden vorzugsweise aus einem gemeinsamen Schlauchelement gebildet. Diese Baugruppe wird vorzugsweise im blow-molding Verfahren hergestellt. Als Material für diese Baugruppe wird vorzugsweise Polyurethan (PUR) verwendet mit chemisch-physikalischen bzw. elastizitäts-mechanisch vergleichbaren Charakteristika wie das Material "Pellethane 2363" der Fa. Dow Chemical Corporation, gefertigt, insbesonder mit der Shore-Härte 80A bis 60D. Neben PUR sind durch blow-molding auch Weichfolien aus PVC und/oder LDPE herstellbar bzw. verarbeitbar.

Bedingt geeignete, voll oder nahezu vollständig auf ihr späteres Arbeitsmaß ausgeformte Ballonelemente können auch im Tauchverfahren hergestellt werden, beispielsweise aus Latex, Silikon oder synthetischen Kautschukarten.

Denkbar ist ebenfalls die Fertigung aus einer oder mehreren Folienlagen der genannten Materialien, die miteinander zu sphärischen Strukturen verklebt und/oder verschweißt werden.

Die Wandstärke der intra-rektalen Ballonhülle 1 sollte im Bereich von 10 bis 40 Mikrometern liegen. Der maximale transversale Durchmesser des intrarektalen Segmentes 1 bei freier, druckloser Entfaltung auf das präformierte Maß sollte bei ca. 6-9 cm liegen. Die axiale Länge des intra-rektalen Ballonkörpers 1 liegt vorzugsweise bei 4-6 cm.

Wird das trans-anale Segment 2 aus separaten Teilen gefertigt, so werden die benötigten dünnwandigen Schlauchfolienlagen ebenfalls bevorzugt durch Blasformung entsprechend dünnwandiger zylindrischer Ballon- bzw. Schlauchkörper hergestellt. Es kann ebenfalls ein Folien-Schweiß- oder Klebeverfahren, wie zuvor beschrieben, angewendet werden. Die verwendeten Materialien sind vorzugsweise mit denen zur Fertigung des intra-rektalen Ankerballons 1 identisch. Wird ein sich elastisch rückstellendes Schlauch- oder Rohrelement 30 integriert, so kann dieses auch aus getauchten oder gespritzten zylinderförmigen Elementen gefertigt werden. PUR der Härten 70A bis 90A bietet die erforderliche selbstaufrichtende Elastizität. Weniger geeignet sind aus Silikon gefertigte Elemente.

Das Trichterelement 3 wird vorzugsweise aus einem Material herstellt, welches mit dem der Ballonhülle 8 identisch ist bzw. eine identische Shore-Härte aufweist. Es wird vorzugsweise im Spritzgußverfahren hergestellt, alternativ ist ein Tauchverfahren denkbar.

Das Material sollte verlässliche elastische Wiederaufrichtungseigenschaften nach passagerer mechanischer Verformung zeigen, und dabei möglichst weich und körperfreundlich erscheinen. Es sollte des Weiteren mit gängigen Lösungsmitteln oder Klebstoffen verklebt werden können. Die geforderten mechanischen und chemischen Eigenschaften werden z.B. von Polyurethanen der Shore-Härten 70 bis 90 gewährleistet. Beispielhaft können Materialen mit physikalisch-chemischen Eigenschaften wie Pellethane der Familie 2363 verwendet werden.

Die Oberfläche des Trichterelements 3 kann zur leichteren Montage desselben an/in dem Ballonelement 8 mit zirkulären Präformierungen auf seiner Oberfläche (sowohl konvex als auch konkav) versehen werden. In axialer Richtung können im Bereich der inneren Oberfläche bzw. des Trichterlumens längs-verlaufende Aussparungen im Sinne von Nuten gefertigt werden, die eventuelle, von proximal durch das trans-anale Segment 2 herangeführte Versorgungsleitungen 32 aufnehmen (Ballonbefüll- bzw. Spüllumina). Ebenfalls können besondere längsverlaufende Kerbungen derart angeordnet werden, dass der Trichter 3 beim Einführen in den Anus durch moderaten manuellen Druck zu einer schlankeren Konfiguration verformt werden kann, und sich im Rektum, durch seine mechanische Rückstellkraft bedingt, spontan in seine ursprüngliche Form aufrichtet.

Das Trichterelement 3 wird bevorzugt olivenförmig, zäpfchenförmig, zigarrenförmig oder torpedoförmig ausgestaltet. Der maximale Außendurchmesser sollte zwischen 4 und 5 cm betragen, das den Stuhl aufnehmende Innenlumen einen Innendurchmesser von 3 cm nicht unterschreiten. Die Länge des Trichters 3 sollte im Bereich von 3 bis 5 cm liegen.

Das Konnektorstück 4 ist an seinem distalen Ende durch entsprechende Nuten, Ringwülste und Durchmesserabstufungen für die montagekompfortable Aufnahme der benötigten jeweiligen trans-analen Elemente 2 ausgelegt. Am proximalen Ende können entsprechende Präformationen zur Aufnahme und Verbindung mit dem vorzugsweise zu einem Beutel ableitenden Drainage- bzw. Entleerungs-Schlauch ausgeformt werden. Das Konnektorstück 4 sollte aus einem verformbaren, sich möglichst selbstaufrichtenden Material gefertigt werden, mit im Wesentlichen dem Trichterelement 3 entsprechenden Eigenschaften.

Der Konnektor 4 kann über spezifische Perforationen bzw. Präformationen einen Ballonbefüllschlauch sowie ein Lumen für die intra-rektale Spülung nach außen hin ableiten. Ebenfalls kann eine nach beutelwärts gerichtete Zuleitung 33 zur Spülung des Drainageschlauchs über den Konnektor 4 hinweg eingeleitet werden.

Der Entleerungsschlauch 5 sollte aus einer dünnwandigen, reißfesten und vorzugsweise nicht bzw. nur wenig dehnbaren Schlauch-Hülle bestehen. Er sollte einen Durchmesser von ca. 2,5 bis 3,5 cm aufweisen und im nicht befüllten Zustand spontan zu einem flachen Band kollabieren bzw. mit moderater Kraft zu einem derartigen Band verformt und als solches sein Inhalt leicht manuell ausstreichbar sein. Seine Länge sollte ca. 20 cm betragen. Eine Wandungsstärke von ca. 0,1 bis 0,2 mm ist hierzu besonders geeignet. Die Länge des entleerenden bzw. drainierenden Schlauches kann bei ca. 1,30 m bis 1,60 m liegen.

Ein erfindungsgemäßer, mit geringer Kraft zu einer bandartigen Struktur kollabierender Entleerungsschlauch 5 kann auch aus verschweißtem Folienmaterial der zuvor genannten PUR-Materialqualitäten hergestellt werden. Ebenfalls kommen beispielweise PVC oder LDPE in Frage.

Im Gegensatz zum bislang vorwiegend verwendeten Basismaterial Silikon weisen PUR-Materialien zudem keine Durchlässigkeit für Gerüche auf. PUR-Materialien verfügen zudem über eine sehr glatte Oberfläche, was die hygienischen Eigenschaften bei prolongierter Lage im Patienten verbessert.

Zur Leerung bzw. Reinigung des Drainageschlauches 5 kann die Vorrichtung eine spangenähnliche, über die gesamte Länge des Drainageschlauchs 5 frei verschiebliche Ausstreichvorrichtung 34 für Stuhl aufweisen.

Die Ausstreichvorrichtung 34 entspricht in der Form vorzugsweise einer Spange, die den Drainageschlauch 5 als verzugsweise geschlossene ringartige Struktur umgibt. Sie erlaubt es, verschiedene Öffnungszustände des Drainagelumens einzustellen. Wird der Schlauch 5 in Position A innerhalb der Spange verschoben, ist das Drainagelumen völlig geöffnet. Befindet sich der Drainageschlauch 5 in Position B, ist sein Lumen derart durch die Backen der Spange 34 verengt, dass der Schlauch 5 zwischen diesen zu einem flachen Band ohne Restlumen zusammengepreßt ist. Eventueller Stuhl im Inneren des Schlauches 5 kann in dieser Spangenposition manuell zu einem Beitel 35 hin verschoben werden.

Die Spange 34 kann ebenfalls zum flüssigkeitsdichten Verschluss des Drainagelumens vorgesehen sein, und kann so als passagerer Verschluss beim Beutelwechsel dienen.

Der Auffangbeutel 35 für Stuhl ist für ca. 1 bis 2 Liter Volumenfassung ausgelegt. Er ist vorzugsweise über ein Konnektorelement 36 - welches ggf. mit einem Schraubgewinde versehen sein kann - mit dem Drainageschlauch 5 verbunden. Das Konnektorelement 36 des Schlauches 5 kann über einen konischen Steck-Anschluß 37 verfügen, der in eine entsprechende Öffnung eines beutel-seitigen Konnektorteils 38 gesteckt wird. Der mit dem Auffangbeutel 35 verbundene Konnektorteil 38 kann mit einem Außengewinde versehen sein. Auf dieses Außengewinde läßt sich eine Überwurfmutter 39 aufschrauben, welche einen an dem Konnektorelement 36 angeformten, rundumlaufenden, in radialer Richtung vorspringenden Bund übergreift und dadurch den konischen Steckanschluß 37 fest in das ggf. mit einem InnenKonus versehene Konnektorteil 38 preßt. Dadurch kann der Schlauch 5 flüssigkeitsdicht mit dem Beutel 35 verbunden werden. Nach dem Wechsel des gefüllten Beutels 35 kann dieser mit einer dem Beutel 35 vorzugsweise über eine flexible Verbindung - bspw. ein Kabel - angefügten Verschlußkappe 40 dicht verschlossen werden.

Wird kein Beutel 35 verwendet, so kann der Steckanschluß 36, 37 mit einem gas-, aber nicht flüssigkeitsdurchlässigen Verschluß 41 im Anwendungsintervall gedichtet werden, wie Fig. 13b zeigt. Der Verschluß 41 kann beispielsweise aus einem porösen Sintermaterial bestehen.

Um beim Beutelwechsel einen Rückstrom von Flüssigkeit aus dem Beutel zu vermeiden, kann das beutelseitige Konnektorteil 38 mit einem Ventilmechanismus versehen sein. Dieser kann - wie in Fig. 13b dargestellt - bspw. aus einem dünnwandigen Schlauchelement 42 bestehen, welches in den Beutel 35 hineinreicht, und dessen Lumen sich nur bei Zufluß von Material in den Beutel hinein öffnet, bei geringfügigem Überdruck im Beutel zur Umgebung hin aber dichtend kollabiert und einen Ausstrom aus dem Beutel 35 vermeidet. Das Schlauchelement 42 hat bevorzugt eine Länge von ca. 5 bis 10 cm.

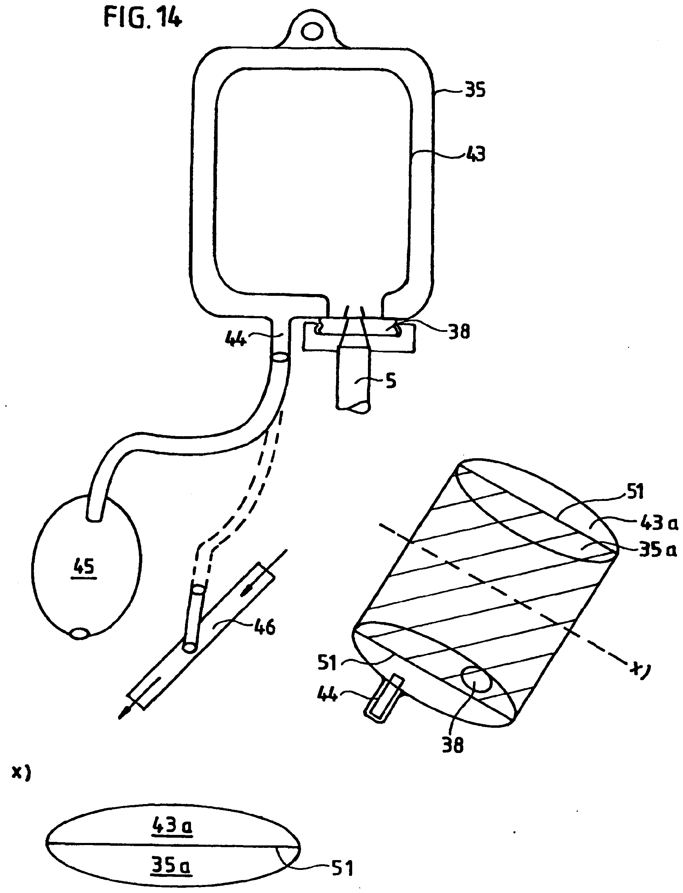

Fig. 14 stellt eine bevorzugte Ausführungsform des Beutels 35 dar. Dieser kann bevorzugt mit einem Innenbeutel 43 versehen sein. Bei dieser Anordnung ist der allseitig vom Beutel 35 umgebene Innenbeutel 43 beispielsweise mit der zuvor beschriebenen Konnektortechnologie 38 ausgestattet, und nimmt entsprechend den Entleerungsschlauch 5 auf. Der Außenbeutel 35 hingegen kann über eine Öffnung 44 mit Druckluft befüllt oder mit einem Vakuum beaufschlagt werden.

Wird der Beutel 35 beispielsweise über eine Handpumpe 45 mit Druckluft befüllt, kann eine zuvor in den Innenbeutel eingebrachte Spüllösung so über den Entleerungsschlauch 5 in das Rektum des Patienten gefördert werden.

Alternativ zum Innenbeutelkonzept, kann der Beutel 35 auch eine separierende Wandung 51 aufweisen, die das Beutelinnere in zwei etwa gleich große, parallel angelegte Kompartimente 35a und 43a unterteilt, wobei im Kompartiment 35a, analog zum Beutel 35 ein Druck aufgebaut werden kann, der zur Ausdehnung des Raumes 35a führt und den Inhalt des Kompartiments 43a analog zum Inhalt des Innenbeutels 43 zum Patienten gerichtet austreibt.

Die beschriebenen Kompartimente 43 und 43a können bereits mit vorgefertigter bzw. speziell zusammengesetzter Spüllösung befüllt sein. Die Schnittebene X verdeutlicht die einander gegenüberliegenden Kompartimente 35a, 43a.

Alternativ kann der Außenbeutel 35 auch über ein an der Öffnung 44 angeschlossenes Vakuum über dem Innenbeutel 43 kollabiert werden und so dessen Füllung in das Rektum austreiben. Das Vakuum kann beispielsweise über eine angeschlossene Wasserstrahlpumpe 46, welche an einem Wasserhahn angeschlossen wird, erzeugt werden.

Setzt die peristaltische Kontraktion des Patienten ein, kann dieser den Druck im Außenbeutel 35 dem Umgebungsdruck angleichen. Die Spüllösung kann dann in den Innenbeutel 43 ablaufen.

Im Anschluss kann die Spüllösung erneut bzw. wiederholt in den Patienten, im Sinne eines Hebe- und Senkeinlaufes, eingebracht werden.

Alternativ kann der Einlauf schwerkraft-getrieben erfolgen. In diesem Falle bringt der Therapeut ein definiertes Volumen von Spüllösung in einem Behälter 35 auf ein geeignet hohes Niveau über dem Patienten. Der Zulauf erfolgt spontan. Die Lösung kann beispielsweise direkt über den Entleerungsschlauch 5 in den Patienten gelangen, oder auch über den Zulauf 32 (siehe Fig. 6) ins Rektum appliziert werden.

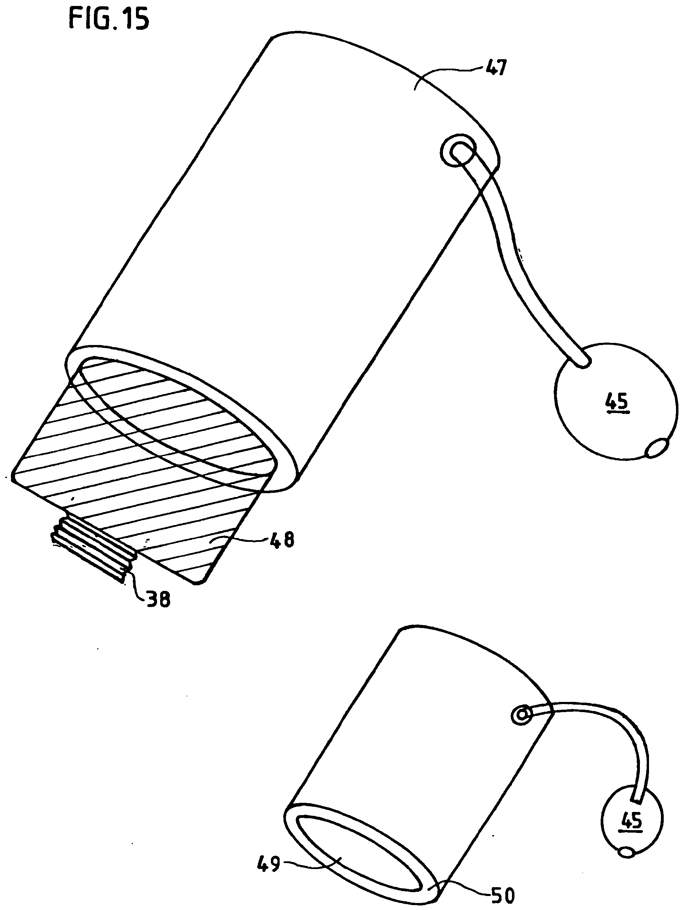

Wie Fig. 15 zeigt, ist zur Entwicklung einer treibenden Kraft für den Einlauf auch eine manschettenartige Vorrichtung 47 denkbar, wie sie beispielsweise für die druck-angetriebene, schnelle intra-vaskuläre Infusion (Druckinfusion) verwendet wird. Ein beutelartiger Behälter 48 mit Spüllösung wird dabei in das offene zentrale Lumen 49 einer mit Druckluft beaufschlagbaren Manschette 50 gesteckt und von dort aus bei steigendem Fülldruck in der Manschette 50 quasi ausgedrückt bzw. zum Patienten hin entleert. Der dazu erforderliche Druck wird bevorzugt mit einer Handpumpe 45 erzeugt. Neben einem gasförmigen Medium zum Druckaufbau in der Manschette 50 sind ebenfalls unter Druck stehende flüssige Medien, wie z.B. Leitungswasser oder über eine angeschlossene Wassersäule denkbar.

Die Verwendung der beschriebenen Druckmanschette 50 ist dann besonders vorteilhaft, wenn verbrauchsfertige, vorgefertigte Lösungen zum Einsatz kommen, die besondere therapeutische oder pflegerische Inhaltsstoffe in den Darm einbringen sollen. Diese können in manschettengerechten Beuteln 48 verpackt sein.

Sich selbst therapierende Patienten können auf der Toilette sitzend abführen, wobei die colo-rektale Spüllösung über den kurzen Entleerungsschlauch direkt in die Toilette abgeleitet werden kann.

Ist ein Verbleiben der Drainagevorrichtung im Intervall zwischen den Spülungen im Patienten nicht gewünscht, kann die beschriebene Einlauftechnik über einen großlumigen Entleerungsschlauch 5, den beschriebenen Beuteln 35, 48 und Manschetten 50 sowie den erfindungsgemäßen Druckerzeugern auch in Kombination mit der in DE 102004033425 A1 und WO 2007/118621 A1 beschriebenen Dichtungs- und Drainagevorrichtung verwendet werden.

Der anal dichtende Ballon 1 bzw. die beschriebenen Ballonanordnungen sind im Stand der Technik auf einem Schaftelement aufgebracht. In Abwandlung zu DE 102004033425 A1 und WO 2007/118621 A1 und im Sinne einer großvolumigen colo-rektalen Spülung zur Erreichung eines kontinenten Intervalls, kann das die Dichtungsvorrichtung tragende Schaftelement einen entsprechend vergrößerten drainierenden Innendurchmesser von ca. 2 bis 3 cm aufweisen. Ferner kann die Länge des ballontragenden Schaftelementes vorteilhaft soweit vergrößert werden, dass der extra-korporale Anteil auch für die bequeme Selbsteinführung des Spülkatheters geeignet ist.

Zwar können die beiden Enden des in sich umgestülpten Schlauchabschnitts 8 etwa koaxial ineinander verlaufen und eventuell mit (je) einer Hülse verbunden sein; bei einer bevorzugten Ausführungsform der Erfindung ist dies jedoch nicht der Fall.

**Bezugszeichenliste**

| | | | |
|---|---|---|---|
| 1 | intrarektaler Ballonabschnitt | 26 | proximales Ende |
| 2 | transanaler Ballonabschnitt | 27 | äußere Hülle,Folienschlauch |
| 3 | Trichterelement | 28 | Kompartiment |
| 4 | Konnektionselement | 29 | Taillierung |
| 5 | Entleerungsschlauch | 30 | Rohrgeflecht |
| 6 | Kompartiment | 31 | Zuleitung |
| 7 | Präformationen | 32 | Zulauf |
| 8 | Ballonfolie | 33 | Spülungslumen |
| 9 | sphärische Formation | 34 | Ausstreichvorrichtung |
| 10 | proximaler Fortsatz | 35 | Auffangbeutel |
| 11 | Trichterloge | 36 | Konnektor |
| 12 | Schlauchrohling | 37 | Steck-Anschluß |
| 13 | Schnittebene | 38 | beutelseitiger Konnektor |
| 14 | ringartige Erhebung | 39 | Überwurfmutter |
| 15 | Spalt | 40 | Verschlußkappe |
| 16 | Ballonhülle | 41 | Verschluß |
| 17 | konkave Präformation | 42 | Schlauchelement |
| 18 | Zwischenraum | 43 | Innenbeutel |
| 19 | Nut | 44 | Öffnung |
| 20 | kanalartige Präformation | 45 | Handpumpe |
| 21 | Trichterkomponente | 46 | Wasserstrahlpumpe |
| 22 | Komponente | 47 | Manschetten-Vorrichtung |
| 23 | ringartiges Element | 48 | beutelartiger Behälter |
| 24 | ringnutartige Präformation | 49 | zentrales Lumen |
| 25 | Rohr- bzw. Schlauchstück | 50 | Manschette |
| | | 51 | Wandung |
| 105 | Ballonenden | 141 | Einheit |
| 106 | Kontaktbereich | 143 | Strebe |
| 107 | unterer Rand | 144 | Bereich |
| 108 | Konnektionselement | 145 | Durchstich |
| 109 | Drainageschlauch | 146 | Spülleitung |
| 110 | Füllschlauch | 147 | Füllleitung |
| 111 | Präformierung | 148 | Ballonkomponente |
| 112 | Spülschlauch | 149 | Kompartiment |
| 113 | Präformierung | 151 | Befestigungspunkt |
| 114 | Präformierung | 152 | Olive |
| 115 | Mündungsöffnung | 153 | Auflagefläche |
| 116 | Ballonrohling | 200 | Fügungsbereich |
| 117 | sphärische Formation | | |
| 118 | Linie | | |
| 119 | schlauchartige Komponente | | |
| 120 | Verstrebung | | |
| 121 | Einheit | | |
| 122 | Rohschlauch | | |
| 123 | Kompartiment | | |
| 124 | Kompartiment | | |
| 125 | Schweißnaht | | |

## Patentansprüche

1. Vorrichtung zum Abdichten einer natürlichen oder künstlichen Öffnung des Dickdarms oder Colons bzw. Enddarms oder Rektums eines Patienten sowie zum Verschließen derselben und/oder zur Entfernung von Stuhl daraus, insbesondere zur intermittierenden Irrigation und/oder zur kontinuierlichen Drainage, vorzugsweise in ein externes, beutelartiges Auffanggefäß, wobei die Vorrichtung
a) untergliedert ist in einen radial erweiterten Bereich als intrarektales Segment (1) zum Einführen in das Rektum sowie einen demgegenüber verjüngten Bereich als transanales Segment (2), der während des Gebrauchs zumindest bereichsweise außerhalb des Rektums verbleibt;
b) einen aufblasbaren Ballon von etwa ringförmiger Struktur umfasst, der aus einem flächigen, in sich umgestülpten Schlauchabschnitt (8) gebildet ist und ein oder mehrere, mit Fluid befüllbare Kompartimente aufweist;
**dadurch gekennzeichnet, dass**
c) das intrarektale Segment (1) ein mit einem Fluid befüllbares, ballonförmiges Kompartiment aufweist,
d) wobei das besagte intrarektale Ballonsegment (1) und das besagte transanale Segment (2) kein gemeinsames Kompartiment besitzen,
e) und wobei das nach distal gerichtete Ende des ausgeformten Ballons (8) durch das Lumen des intrarektalen Ballonsegmentes (1) gestülpt und im Lumen des nach proximal gerichteten Ballonendes weitergeführt ist und im Bereich des proximalen Ballonendes mit jenem verbunden ist, um das befüllbare Kompartiment des intrarektalen Ballonsegmentes (1) abzuschließen.

2. Vorrichtung nach Anspruch 1, **dadurch gekennzeichnet, dass** das Basismaterial für den Ballon bzw. für die Ballondichtung ein Material mit einer derart geringen Volumendehnbarkeit ist, dass sich der Ballon unter anliegender Zugwirkung nicht nach distal tropfenförmig ausstreicht und durch die Analöffnung schlüpft, bevorzugt Polyurethan der Spezifikation Pellethane 2363 80A der Dow Chemical Corp., wobei insbesondere das intra-rektale und/oder das trans-anale Segment (1,2) aus Polyurethan gefertigt ist.

3. Vorrichtung nach einem der vorhergehenden Ansprüche, **dadurch gekennzeichnet, dass** das trans-anale Drainage-Lumen mit einem Durchmesser von bis zu 3 cm ausgeformt ist, um den freien Abgang auch geformter Stuhlanteile zu gestatten.

4. Vorrichtung nach einem der vorhergehenden Ansprüche, **dadurch gekennzeichnet, dass** das trans-anale Segment (2) der Vorrichtung bereichsweise ein-lagig ausgeführt ist.

5. Vorrichtung nach einem der vorhergehenden Ansprüche, **dadurch gekennzeichnet, dass** das trans-anale Segment (2) der Vorrichtung derart ausgebildet ist, dass es sich ohne äußere Krafteinwirkung, also z. B. im frei entfalteten Zustand, ohne dem Analkanal anzuliegen, durch seine elastische Kraft in seinem Querschnitt kreisrund aufrichtet.

6. Vorrichtung nach einem der vorhergehenden Ansprüche, **dadurch gekennzeichnet, dass** das trans-anale Segment (2) neben einer ein- oder zwei-lagigen Schlauchlage ein zusätzliches funktionelles Element aufweist, welches einer axialen Torsion bzw. dem verwindungsbedingten Verschluss dieses Abschnittes entgegenwirken soll.

7. Vorrichtung nach einem der vorhergehenden Ansprüche, **dadurch gekennzeichnet, dass** die äußere Schlauchlage des trans-analen Segments (2) ein dünnwandiges, zylindrisches oder dem Analkanal kongruent präformiert ausgeformtes Foienschlauchelement ist.

8. Vorrichtung nach einem der vorhergehenden Ansprüche, **gekennzeichnet durch** ein Element zum passageren Verschluss des Drainagelumens im Bereich des trans-analen Segments (2).

9. Vorrichtung nach einem der vorhergehenden Ansprüche, **dadurch gekennzeichnet, dass** das intra-rektale Ballonsegment residual ausgeformt ist, also über die der jeweiligen Anatomie folgenden Maße hinaus präformiert ist, wobei das initial eingebrachte Füllvolumen bei 70 bis 80 % des frei entfalteten, präformierten Volumens liegt, so dass der Ballonkörper dem Rektumboden als schlaffer, nur teilweise gefüllter Ballonkörper aufliegt.

10. Vorrichtung nach einem der vorhergehenden Ansprüche, **dadurch gekennzeichnet, dass** der Ballon des intra-rektalen Segments (1) im rückgestülpten, montierten Zustand eine logenartige Präformation (11) zur Aufnahme eines separat gefertigten Trichterelementes (3) aufweist.

11. Vorrichtung nach Anspruch 10 in Verbindung mit Anspruch 11, **gekennzeichnet durch** ein das Trichterelement (3) mit dem Konnektor (4) zu einer vorzugsweise durchgängigen Einheit (141) verbindendes Rohrelement (25) zur Stabilisierung des trans-analen Segmentes (2), wobei die Einheit (141) selbstaufrichtende Eigenschaften hat.

12. Vorrichtung nach einem der vorhergehenden Ansprüche, **dadurch gekennzeichnet, dass** im Bereich des proximalen Endes des intrarektalen Ballons (1) eine direkte Verbindung der Ballonhülle mit der Oberfläche von Anteilen des trans-analen Segmentes (2) hergestellt ist oder indirekt über ein Trichterelement (3).

13. Vorrichtung nach einem der Ansprüche 11 bis 13, **dadurch gekennzeichnet, dass** die Trennung der beiden Segmente (1,2) durch einen dauerhaften, dichtenden Abschluss der Hülle des intra-rektalen Ballonsegmentes (1) auf oder mit einem Trichterelement (3) bewirkt wird.

14. Vorrichtung nach einem der vorhergehenden Ansprüche, **dadurch gekennzeichnet, dass** die Trennung der beiden Segmente (1,2) im Bereich des proximalen Endes des intra-rektalen Ballons (1) durch direkte Verbindung der Ballonhülle mit der Oberfläche von Anteilen des trans-analen Segmentes (2) hergestellt ist.

15. Vorrichtung nach einem der Ansprüche 1 bis 14, **dadurch gekennzeichnet, dass** das transanale Segment (2) aus einem einzigen Schlauchelement hergestellt ist, insbesondere aus einem einzigen Ballon- oder Folienschlauchkörper.

16. Vorrichtung nach einem der Ansprüche 1 bis 14, **dadurch gekennzeichnet, dass** das transanale Segment (2) aus mehreren, separat gefertigten Schlauchelementen hergestellt ist, insbesondere aus mehreren, separat hergestellten Ballon- oder Folienschlauchkörpern.

17. Vorrichtung nach einem der vorhergehenden Ansprüche, **gekennzeichnet durch** einen angeschlossenen Drainageschlauch (5), insbesondere einen kurzen, tüllenartigen Schlauch oder Folienschlauch zur Ableitung ins Toilettenbecken.
